# EUROPEAN PATENT APPLICATION

(11) **EP 4 169 931 A1**
(43) Date of publication of application: **26.04.2023**
(21) Application number: 21828968.4
(22) Date of filing: 21.06.2021
(51) Int. Cl.: C07H 21/00, C12N 9/99, C12P 19/34

(54) **NUCLEIC ACID LIGAND AND USE THEREOF**

(30) Priority: 22.06.2020 CN 202010576818
(71) Applicant: Nuhigh Biotechnologies Co., Ltd, Suzhou, Jiangsu 215123 (CN)
(72) Inventor: BI, Wanli, Suzhou, Jiangsu 215123 (CN); HE, Wenlong, Suzhou, Jiangsu 215123 (CN); FU, Dongke, Suzhou, Jiangsu 215123 (CN); PAN, Jie, Suzhou, Jiangsu 215123 (CN); XING, Yadong, Suzhou, Jiangsu 215123 (CN); WANG, Zhiqing, Suzhou, Jiangsu 215123 (CN); QIN, Pingping, Suzhou, Jiangsu 215123 (CN)
(74) Representative: Gill Jennings & Every LLP
(86) International application number: PCT/CN2021/101244
(87) International publication number: WO 2021/259201

(57) **Abstract**

Provided are a nucleic acid ligand (nucleic acid polymerase substrate analog), a mixture thereof, and the use thereof. The mixture of the nucleic acid polymerase substrate analog contains two or more nucleic acid polymerase substrate analogs. The nucleic acid polymerase substrate analog is a single nucleic acid molecule or nucleic acid molecule analog which forms complementary pairing within a molecule, or a single or two nucleic acid molecules or nucleic acid molecule analogs which form complementary pairing between molecules; and a structure formed thereby has the characteristics of a nucleic acid polymerase substrate. When an amplification reaction mixture is at or below a certain temperature, the enzyme activity of a nucleic acid polymerase is inhibited by means of the nucleic acid ligand and there is no residual enzyme activity. When the reaction mixture is heated, the nucleic acid polymerase is separated from the nucleic acid polymerase substrate analog to exert activity and form a primer extension product, thereby achieving the effect of inhibiting non-specific amplification. The nucleic acid polymerase substrate analog is suitable for all polymerases and can be widely used in the field of nucleic acid amplification. The 3' end of the nucleic acid ligand has a modification which inhibits the extension thereof.

## Description

### TECHNICAL FIELD

The present invention relates to the field of biotechnology, and more particularly to a nucleic acid ligand (nucleic acid polymerase substrate analog) and the use thereof, and a mixture of the nucleic acid polymerase substrate analogs and the use thereof.

### BACKGROUND OF THE INVENTION

Polymerase chain reaction (PCR) is a molecular biological technique used to amplify specific DNA fragments. It is a special DNA replication in vitro, which can greatly increase the trace amount of DNA. PCR consists of three basic reaction steps: denaturation-annealing-extension. ① denaturation of a template DNA: After a template DNA is heated to about 93 °C for a certain period of time, the double strands of the template DNA or the double-stranded DNA formed by PCR amplification is dissociated to form a single strand, which binds to primers for the next round of reaction; ② annealing of the template DNA with primers (i.e., renaturation): after the template DNA is heated and denatured into single strands, the temperature is reduced to about 55°C, and the primers are paired with the complementary sequence of the template DNA single strand; ③ extension of primers: Under the action of DNA polymerase (such as Taq DNA polymerase) at 72°C, using dNTP as the starting material and the target sequence as the template, DNA template-primer combination is used to synthesize a new semi-conservative replication strand complementary to the template DNA strand according to the principle of base complementary pairing and semi-conservative replication. Repeating the three processes of denaturation-annealing-extension can result in more "semi-conservative replication strand", and this new strand can act as the template for the next cycle. It takes 2-4 minutes to complete each cycle, and it takes 2 to 3 hours to amplify the gene of interest to be amplified by millions to billions of times.

Although PCR has been widely used in the field of biomedicine, non-specific amplification due to PCR side reactions often causes major problems. Particularly in the field of clinical diagnostics, it is necessary to amplify trace amounts of DNA of interest among a large amount of background DNA, where the non-specific amplification may result in false positives. The main cause for non-specific amplification is that the enzyme allows the primers which anneal non-specifically to extend at room temperature. Thus, inhibiting the activity of polymerase at room temperature can greatly reduce non-specific amplification.

In order to reduce or even avoid the occurrence of non-specific amplification in the process of operation, a hot-start polymerase chain reaction has been invented, and a hot-start polymerase has been developed and used. The hot-start enzyme can avoid mismatch in the system at low temperature by means of hot start, and the principle is to block the activity centre of the enzyme by chemical modification or antibody modification. In the chemical modification, some molecular groups are used to bind to the activity centre of the enzyme, and when the temperature reaches a certain temperature (generally before the annealing temperature), small molecules leave the activity centre of the enzyme, and the activity centre of the enzyme is exposed to exert activity and guide the amplification of the system; however, the performance of chemically modified hot-start enzymes is unstable. In antibody modification, a modified polymerase is used as antigen to immunize experimental animals to produce the corresponding antibody, and after a series of screening, the antibody is prepared on a large scale by monoclonal antibody technology. Then, the antibody is inactivated and shed at high temperature in the PCR reaction by using the biological activity of the antibody to achieve the effect of hot start; however, the production of specific antibodies needs a very long screening period, and the antibody modification tends to bring about the contamination of exogenous DNA. Antibodies dissociate from polymerases generally at high temperatures and therefore are unsuitable for polymerase intolerant to high temperatures such as reverse transcriptase.

United States patents (US6183967, US6020130) disclose oligonucleotide aptamers that bind specifically to thermostable Taq enzymes, Tth enzymes, and TZ05 enzymes, and these aptamers can block polymerase activity at room temperature. The process of screening for aptamers generally comprises five basic steps, namely: binding, separation, elution, amplification and modulation, and then the target aptamers are obtained through iteration cycle. The entire screening process requires very long cycles, which is relatively slow and complex. And the aptamers screened by a particular method are highly specific for the corresponding ligand (polymerase), and thus different aptamers are required for different polymerases.

Thus, there is a need for a more convenient method for reversibly inhibiting a nucleic acid polymerase so that the nucleic acid ligands (nucleic acid polymerase substrate analogs) that inhibit the enzyme activity of nucleic acid polymerases can be used more universally and are suitable for more types of nucleic acid polymerases. The nucleic acid ligands (nucleic acid polymerase substrate analogs) and mixtures thereof according to the present invention more effectively inhibit the enzyme activity of nucleic acid polymerases at a certain temperature.

### SUMMARY OF THE INVENTION

In view of this, the purpose of the present invention is to provide a nucleic acid ligand (nucleic acid polymerase substrate analog) and a mixture thereof, which are capable of effectively reducing non-specific amplification products caused by a nucleic acid polymerase at room temperature and are suitable for all types of nucleic acid polymerases with greater universality.

The nucleic acid polymerase substrate analog according to the present invention binds to a nucleic acid polymerase by mimicking the substrate which binds to the nucleic acid polymerase, and are therefore referred to as a nucleic acid polymerase substrate analog. It can make nucleic acid polymerases lose or regain activity under the control of temperature. The nucleic acid polymerase substrate analog can be suitable for all nucleic acid polymerases.

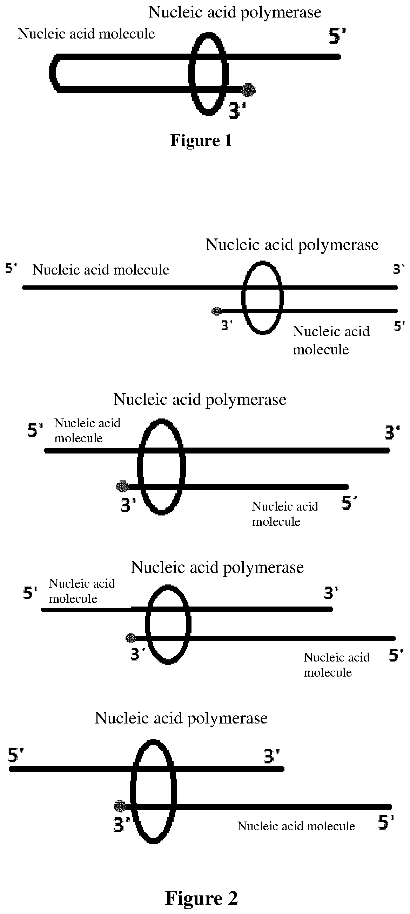

Wherein, where said nucleic acid polymerase substrate analog is a single nucleic acid molecule or a nucleic acid molecule analog forming intramolecular complementary pairing, the schematic diagram of the interaction with a nucleic acid polymerase is shown in Figure 1; where the nucleic acid polymerase substrate analog is a single or two nucleic acid molecules or nucleic acid molecule analogs that form intermolecular complementary pairing, the schematic diagram is shown in Figure 2.

Another purpose of the present invention is to provide use of the above nucleic acid ligand (nucleic acid polymerase substrate analog) in nucleic acid amplification, and a mixture thereof, in the preparation of a nucleic acid amplification kit and in the preparation of a nucleic acid extension reaction mixture;

Another purpose of the present invention is to provide a method of nucleic acid amplification, in which the nucleic acid polymerase substrate analog described above or a mixture thereof to amplify a target nucleic acid in a test sample;

Another purpose of the present invention is to provide a nucleic acid amplification kit and a nucleic acid extension reaction mixture comprising the above nucleic acid ligand (nucleic acid polymerase substrate analog) or a mixture thereof;

For realizing the above-mentioned purpose of the invention, the present invention provides the following technical solutions:
The nucleic acid ligand according to the present invention is a single nucleic acid molecule or nucleic acid molecule analog which forms intramolecular complementary pairing, or a single or two nucleic acid molecules or nucleic acid molecule analogs which form intermolecular complementary pairing; said nucleic acid ligand is modified at 3' end, which inhibits its extension, and forms a stable structure with a nucleic acid polymerase when the temperature is maintained or below a certain temperature, and the enzyme activity of the nucleic acid polymerase is inhibited at this time; when the temperature is higher than said certain temperature, the nucleic acid polymerase detaches from said nucleic acid ligand (nucleic acid polymerase substrate analog) and exert its activity.

The nucleic acid ligand (nucleic acid polymerase substrate analog) of the present invention binds to a nucleic acid polymerase by mimicking the substrate which binds to the nucleic acid polymerase, and thus may also be referred to as a nucleic acid polymerase substrate analog. It can make nucleic acid polymerases lose or regain activity under the control of temperature. Said nucleic acid ligand (nucleic acid polymerase substrate analog) can be suitable for all nucleic acid polymerases.

Wherein, where said nucleic acid ligand (nucleic acid polymerase substrate analog) is a single nucleic acid molecule or a nucleic acid molecule analog forming intramolecular complementary pairing, the schematic diagram of the interaction with a nucleic acid polymerase is shown in Figure 1; where said nucleic acid ligand (nucleic acid polymerase substrate analog) is a single or two nucleic acid molecules or nucleic acid molecule analogs that form intermolecular complementary pairing, the schematic diagram is shown in Figure 2.

Preferably, the certain temperature is a temperature at which the nucleic acid polymerase exerts its activity; in a specific embodiment of the present invention, the certain temperature should be significantly lower than the reaction temperature. For example, for a thermostable DNA polymerase used in PCR, the temperature is 50°C.

Preferably, the number of the complementary pairing is 8-35, or 10-30, or 10-20; in a specific embodiment of the present invention, the number of the intramolecular complementary pairing is 8-20 and the number of the intermolecular complementary pairing is 10-32.

Preferably, the nucleic acid molecule or nucleic acid molecule analog is modified at its 3' end, which inhibits its extension, and the modification includes the modification of dideoxygenation, or phosphorylation, or amino modification and the like.

In another aspect, the invention provides a mixture of nucleic acid ligands (nucleic acid polymerase substrate analogs), wherein:
a. containing two or more nucleic acid polymerase substrate analogs;
b. the nucleic acid polymerase substrate analog is a single oligomeric nucleic acid molecule or nucleic acid molecule analog which forms intramolecular complementary pairing, or a single or two oligomeric nucleic acid molecules or nucleic acid molecule analogs which form intermolecular complementary pairing; the nucleic acid polymerase substrate analog forms a structure which has the characteristics of a nucleic acid polymerase substrate;
c. the nucleic acid polymerase substrate analogs are modified at 3' end, which inhibits their extension;
d. the two or more nucleic acid polymerase substrate analogs have different widths of temperature adaptation range;
e. when the temperature is maintained at or below a first temperature, the two or more nucleic acid polymerase substrate analogs are mixed with a nucleic acid polymerase and the two form a nucleic acid polymerase-substrate analog complex; at this time, the enzyme activity of the nucleic acid polymerase is significantly reduced relative to that in the absence of the nucleic acid polymerase substrate analog;
f. when the temperature is higher than the first temperature, the nucleic acid polymerase-substrate analog complex described in "e" disintegrates, and all or part of the nucleic acid polymerase activity is released.

The invention also provides a mixture of a nucleic acid polymerase and a mixture of nucleic acid ligands (nucleic acid polymerase substrate analogs), wherein:
a. containing two or more nucleic acid polymerase substrate analogs;
b. the nucleic acid polymerase substrate analog is a single oligomeric nucleic acid molecule or nucleic acid molecule analog which forms intramolecular complementary pairing, or a single or two oligomeric nucleic acid molecules or nucleic acid molecule analogs which form intermolecular complementary pairing; the nucleic acid polymerase substrate analog forms a structure which has the characteristics of a nucleic acid polymerase substrate, and can bind to a nucleic acid polymerase; the molecule number of each nucleic acid polymerase substrate analog is greater than the molecule number of the nucleic acid polymerase, i.e. the molar concentration of each nucleic acid polymerase substrate analog is greater than the molar concentration of the nucleic acid polymerase;
c. the nucleic acid polymerase substrate analogs are modified at 3' end, which inhibits their extension;
d. the two or more nucleic acid polymerase substrate analogs have different widths of temperature adaptation range;
e. when the temperature is maintained at or below the first temperature, the two or more nucleic acid polymerase substrate analogs are mixed with a nucleic acid polymerase and the two form a nucleic acid polymerase-substrate analog complex; at this time, the enzyme activity of the nucleic acid polymerase is significantly reduced relative to that in the absence of the nucleic acid polymerase substrate analog;
f. when the temperature is higher than the first temperature, the nucleic acid polymerase-substrate analog complex described in "e" disintegrates, and all or part of the nucleic acid polymerase activity is released.

In a preferred embodiment of the invention, g. when the temperature is maintained at or below a second temperature, the nucleic acid polymerase substrate analog with a wide temperature adaptation range and the nucleic acid polymerase form a nucleic acid polymerase-substrate analog complex, and the nucleic acid polymerase substrate with a narrow temperature adaptation range cannot form a nucleic acid polymerase-substrate analog complex with the nucleic acid polymerase;

The first temperature is higher than the second temperature.

According to the present invention, the two or more nucleic acid polymerase substrate analogs have different widths of temperature adaptation range. When the temperature is maintained at or below the first temperature, the two or more nucleic acid polymerase substrate analogs form a stable structure with the nucleic acid polymerase, and the enzyme activity of the nucleic acid polymerase is inhibited at this time; when the temperature is maintained at or below the second temperature (below the first temperature), the nucleic acid polymerase substrate analog with a wide temperature adaptation range forms a stable structure with the nucleic acid polymerase, but the nucleic acid polymerase substrate analog with a narrow temperature adaptation range cannot form a stable structure with the nucleic acid polymerase, i.e. the nucleic acid polymerase substrate analog with a narrow temperature adaptation range has lost the ability to inhibit the nucleic acid polymerase, and the enzyme activity of the nucleic acid polymerase is inhibited only by another more stable nucleic acid polymerase substrate analog; when the temperature is higher than the first temperature, the two or more nucleic acid polymerases detach from the nucleic acid polymerase substrate analog to exert activity.

According to the present invention, the width of temperature adaptation range refers to the temperature range in which the nucleic acid polymerase substrate analogs can form a stable structure with the nucleic acid polymerase (e.g. 2-70°C, 5-65 °C, etc.); the nucleic acid polymerase substrate analog with a wide temperature adaptation range refers to a nucleic acid polymerase substrate analog that is capable of forming a stable structure with the nucleic acid polymerase at both the first temperature and the second temperature; a nucleic acid polymerase substrate analog with a narrow temperature adaptation range refers to a nucleic acid polymerase substrate analog that is capable of forming a stable structure with a nucleic acid polymerase at the first temperature, but is incapable of forming a stable structure with a nucleic acid polymerase at the second temperature.

The nucleic acid polymerase substrate analog of the present invention binds to a nucleic acid polymerase by mimicking substrates which bind to the nucleic acid polymerase, such as NTP (nucleoside triphosphate) or dNTP (deoxyribonucleoside triphosphate). It can make nucleic acid polymerases lose or regain activity under the control of temperature. The nucleic acid polymerase substrate analog can be suitable for all nucleic acid polymerases.

In a preferred embodiment of the present invention, there is a temperature difference between the first temperature and the second temperature, which is greater than or equal to 5°C.

By way of example, the specific interaction between the mixture of nucleic acid polymerase substrate analogs of the present invention and the nucleic acid polymerase is set forth as follows:
Where the mixture of nucleic acid polymerase substrate analogs contains two nucleic acid polymerase substrate analogs, i.e., a first nucleic acid polymerase substrate analog and a second nucleic acid polymerase substrate analog, respectively; the first temperature is 50°C and the second temperature is 4°C: When the temperature is maintained at or below 50°C, the two nucleic acid polymerase substrate analogs can both form a stable structure with the nucleic acid polymerase, and the enzyme activity of the nucleic acid polymerase is inhibited at this time; when the temperature is maintained at or below 4°C, the more stable nucleic acid polymerase substrate analog forms a stable structure with the nucleic acid polymerase, but the more unstable nucleic acid polymerase substrate analog cannot form a stable structure with the nucleic acid polymerase, i.e. the more unstable nucleic acid polymerase substrate analog has lost the ability to inhibit the nucleic acid polymerase and the enzyme activity of the nucleic acid polymerase is inhibited only by another more stable nucleic acid polymerase substrate analog; when the temperature is higher than 50°C, the two or more nucleic acid polymerases detach from the nucleic acid polymerase substrate analog to exert activity.

The inventors have found that the width of the temperature adaptation range of the nucleic acid polymerase substrate analog is related to the number of its intramolecular or intermolecular complementary paired bases, i.e., in the mixture of the nucleic acid polymerase substrate analogs of the present invention, the two or more nucleic acid polymerase substrate analogs have different numbers of intramolecular or intermolecular complementary paired bases. When the temperature is maintained at or below the second temperature, the nucleic acid polymerase substrate analog having less complementary paired bases forms a stable structure with the nucleic acid polymerase, but the nucleic acid polymerase substrate analog having more complementary paired bases cannot form a stable structure with the nucleic acid polymerase, that is, the nucleic acid polymerase substrate analog having more complementary paired bases has lost the ability to inhibit the nucleic acid polymerase, and at this time the enzyme activity of the nucleic acid polymerase is inhibited only by the nucleic acid polymerase substrate analog having less complementary paired bases. However, when the temperature is maintained at or below the first temperature but above the second temperature, the two or more nucleic acid polymerase substrate analogs can form a stable structure with the nucleic acid polymerase and inhibit the activity of the nucleic acid polymerase.

The present inventors have investigated the inhibition of the number of intramolecular or intermolecular complementary paired bases on enzyme activity, and found that as the number of paired bases increases, the inhibition on enzyme activity increases gradually and later decreases. Thus, in a preferred embodiment of the invention, the number of complementary paired bases is 8-35;
preferably, the number of complementary paired bases is 10-30;
more preferably, the number of complementary paired bases is 10-20;
further preferably, the number of intramolecular complementary paired bases is 8-20, and the number of intermolecular complementary paired bases is 10-32.

Since the width of the temperature adaptation range of the nucleic acid polymerase substrate analog is related to the number of its intramolecular or intermolecular complementary paired bases and has little correlation with its own nucleic acid sequence, the present invention uses different widths of the temperature adaptation ranges of the nucleic acid polymerase substrate analogs at different temperatures to achieve the inhibition on the enzyme activity at a certain temperature (especially at a low temperature), thereby achieving the inhibition on non-specific amplification. Therefore, based on the mechanism of interaction between the nucleic acid polymerase substrate analogs of the present invention and the nucleic acid polymerase, the nucleic acid sequence of the nucleic acid polymerase substrate analog is not specifically defined in the present invention, as long as the number of complementary paired bases in the nucleic acid polymerase substrate analogs satisfies the conditions of the present invention, and the numbers of intramolecular or intermolecular complementary paired bases for the two or more nucleic acid polymerase substrate analogs are different. The present invention also verifies in the examples that the addition of one, two, three or four nucleic acid polymerase substrate analogs to one nucleic acid polymerase substrate analog can effectively inhibit the non-specific amplification at a certain temperature (especially at a low temperature), i.e. that the inhibition on the non-specific amplification according to the present invention has little correlation with the sequences of the nucleic acid polymerase substrate analogs themselves, and mainly depends on the number of their intramolecular or intermolecular complementary paired bases.

In a preferred embodiment of the invention, the nucleic acid polymerase substrate analog has a 3' end that is a non-OH group; the principle is that any -OH group at the 3' end is capable of forming a complex with a nucleic acid polymerase; modifications at the 3' end of a nucleic acid polymerase substrate analog that inhibit its extension include, but are not limited to, the modification of dideoxygenation, or phosphorylation, or amino modification, and the like.

The present inventors utilize a dideoxy method to modify the 3' end to stop terminal extension, meanwhile use a nucleic acid molecule that is not modified at the 3' end as control, to test whether a nucleic acid molecule that is not modified at the 3' end can also inhibit enzyme activity. The results showed that when the control nucleic acid ligand was added, the activity of the enzyme kept full activity and increased rapidly; while in the system with the addition of the modified nucleic acid ligand, the enzyme activity is largely inhibited to achieve the desired result and the system can be blocked. This assay demonstrates that modification at the 3' end of a nucleic acid ligand is important for inhibiting the enzyme activity of nucleases.

The nucleic acid ligands (nucleic acid polymerase substrate analogs) of the invention and mixtures thereof are suitable for all polymerases, including a DNA polymerase and a RNA polymerase. The DNA polymerase is a thermostable DNA polymerase such as from Family A, for example, Thermus aquaticus, Thermus thermophilus, Thermus filiformis, Thermus flavu, Bacillus stearothermophilus and the like, and from Family B, for example, Pyrococcus furiosus, Thermococcus Kodakaraensis and the like, and the RNA polymerase is a reverse transcriptase from the AMV family, MMLV family and the like. The examples of the present invention also demonstrate that a mixture of nucleic acid polymerase substrate analogs can inhibit the activity of a reverse transcriptase or DNA polymerase at a certain temperature, and when the temperature is higher than a certain temperature, the activity of the nucleic acid polymerase is partially or completely released. This indicates that the mixture of the nucleic acid polymerase substrate analogs according to the present invention can inhibit the enzyme activity of all types of nucleic acid polymerases at a certain temperature, and is suitable for all types of nucleic acid polymerases with greater universality.

Amplification of a target gene with a nucleic acid ligand of the invention (nucleic acid polymerase substrate analog) is capable of significantly inhibiting non-specific amplification compared to a control without the addition of the nucleic acid ligand (nucleic acid polymerase substrate analog). Therefore, the invention also provides use of the nucleic acid ligand (nucleic acid polymerase substrate analog) in nucleic acid amplification, in the preparation of a nucleic acid amplification kit or in the preparation of a nucleic acid extension reaction mixture.

The mixture of nucleic acid polymerase substrate analogs according to the present invention comprises two or more nucleic acid polymerase substrate analogs, and at least two nucleic acid polymerase substrate analogs have different widths of temperature adaptation range to better inhibit the enzyme activity of the nucleic acid polymerase at a certain temperature compared to a control to which one nucleic acid polymerase substrate analog is added, thereby better inhibiting non-specific amplification. The invention therefore also provides use of the mixture of nucleic acid polymerase substrate analogs in nucleic acid amplification, in the preparation of a nucleic acid amplification kit or in the preparation of a nucleic acid extension reaction mixture.

According to the above application, the present invention provides a method of nucleic acid amplification, comprising:
Step 1: contacting a sample containing a target nucleic acid to be tested with the following amplification reaction reagents to form a reaction mixture;
   a) primers that can hybridize to the target nucleic acid;
   b) a nucleic acid polymerase;
   c) the nucleic acid ligand (nucleic acid polymerase substrate analog) of the present invention;
   d) a nucleoside triphosphate;
Step 2: heating the reaction mixture to allow the paired nucleotides of the nucleic acid ligand (nucleic acid polymerase substrate analog) to dissociate into a single strand and the nucleic acid polymerase to detach from the nucleic acid ligand (nucleic acid polymerase substrate analog) and exert its activity, thereby forming a primer extension product.

Preferably, the nucleoside triphosphate includes dUTP, dATP, dCTP, dGTP, dTTP.

Preferably, the method of amplifying a target nucleic acid in a sample to be tested further comprises detecting the primer extension product.

According to the above application, the present invention provides a method of nucleic acid amplification, comprising:
Step 1: contacting a sample containing a target nucleic acid to be tested with the following amplification reaction reagents to form a reaction mixture;
   a) primers that can hybridize to the target nucleic acid;
   b) a nucleic acid polymerase;
   c) a mixture of nucleic acid polymerase substrate analogs;
   d) a nucleoside triphosphate, a deoxynucleoside triphosphate or a mixture thereof, or a nucleoside/deoxynucleoside triphosphate analog;
Step 2: heating the reaction mixture to allow the paired nucleotides of the nucleic acid polymerase substrate analog to dissociate into a single strand and the nucleic acid polymerase to detach from the nucleic acid polymerase substrate analog and exert its activity, thereby forming a primer extension product.

In a preferred embodiment, the nucleoside triphosphate includes dUTP, dATP, dCTP, dGTP, or dTTP.

In a preferred embodiment, the method of nucleic acid amplification further comprises detecting the primer extension product.

In addition, the present invention also provides a nucleic acid amplification kit, comprising the nucleic acid ligands (nucleic acid polymerase substrate analogs) of the present invention. Meanwhile, the present invention also provides a nucleic acid extension reaction mixture comprising the nucleic acid ligands (nucleic acid polymerase substrate analogs) of the invention, a nucleic acid polymerase, at least one primer, a nucleic acid template, and a nucleoside triphosphate.

In addition, the present invention provides a nucleic acid amplification kit comprising the above-mentioned mixture of the nucleic acid polymerase substrate analogs or the above-mentioned mixture of the nucleic acid polymerase and the mixture of nucleic acid polymerase substrate analogs.

Meanwhile, the present invention also provides a nucleic acid extension reaction mixture comprising the above-mentioned mixture of the nucleic acid polymerase substrate analogs or the mixture comprising the above-mentioned mixture of the nucleic acid polymerase substrate analogs and a nucleic acid polymerase, optionally a nucleic acid polymerase, at least one primer, a nucleic acid template; and a nucleoside triphosphate, a deoxyribonucleoside triphosphate or a mixture of both, or a nucleoside triphosphate/deoxyribonucleoside triphosphate analog.

It can be seen from the above technical solutions that the present invention provides a nucleic acid ligand (nucleic acid polymerase substrate analog): when the temperature of the amplification reaction mixture is maintained at or below a certain temperature, the enzyme activity of the nucleic acid polymerase is inhibited by the nucleic acid ligand (nucleic acid polymerase substrate analog) without residual enzyme activity; when the reaction mixture is heated, the nucleic acid polymerase detaches from the nucleic acid ligand (nucleic acid polymerase substrate analog) to exert its activity, thereby forming a primer extension product and achieving the effect of inhibiting non-specific amplification. The nucleic acid ligand (nucleic acid polymerase substrate analog) of the present invention is suitable for all polymerases and can be widely used in the field of nucleic acid amplification, thereby reducing non-specific amplification.

The present invention provides a mixture of nucleic acid polymerase substrate analogs: when the temperature of the amplification reaction mixture is maintained at or below a certain temperature, the enzyme activity of the nucleic acid polymerase is inhibited by the mixture of nucleic acid polymerase substrate analogs without residual enzyme activity; when the reaction mixture is heated, the nucleic acid polymerase detaches from the mixture of nucleic acid polymerase substrate analogs to exert its activity, thereby forming a primer extension product and achieving the effect of inhibiting non-specific amplification. The mixture of nucleic acid polymerase substrate analogs of the present invention is suitable for all polymerases and can be widely used in the field of nucleic acid amplification, thereby reducing non-specific amplification.

### BRIEF DESCRIPTION OF THE DRAWINGS

Figure 1 shows a schematic diagram of the interaction of a nucleic acid ligand (nucleic acid polymerase substrate analog) (intramolecular complementary pairing) of the invention with a nucleic acid polymerase;
Figure 2 shows a schematic diagram of the interaction of a nucleic acid ligand (nucleic acid polymerase substrate analog) (intermolecular complementary pairing) of the invention with a nucleic acid polymerase;

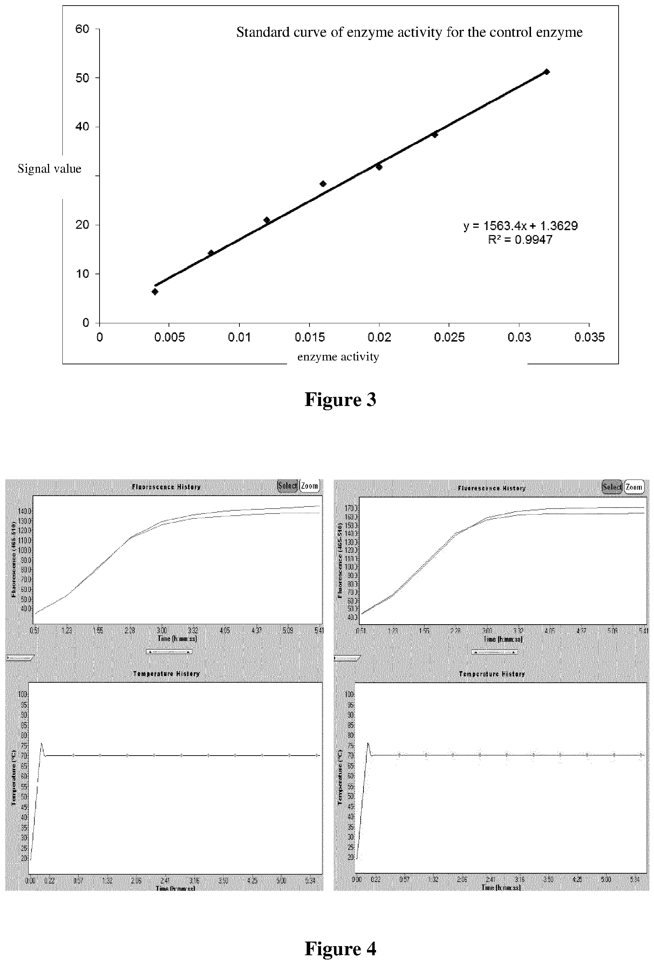

Figure 3 shows a standard curve diagram of the enzyme activity of the control enzyme in the test method;
Figure 4 shows the results of isothermal extension amplification at 70 °C; wherein the left graph shows the case where the nucleic acid ligand (nucleic acid polymerase substrate analog) is added (the upper shows an amplification curve and the lower shows a reaction temperature), and the right graph shows the case where no nucleic acid ligand is added (the upper shows an amplification curve and the lower shows a reaction temperature);

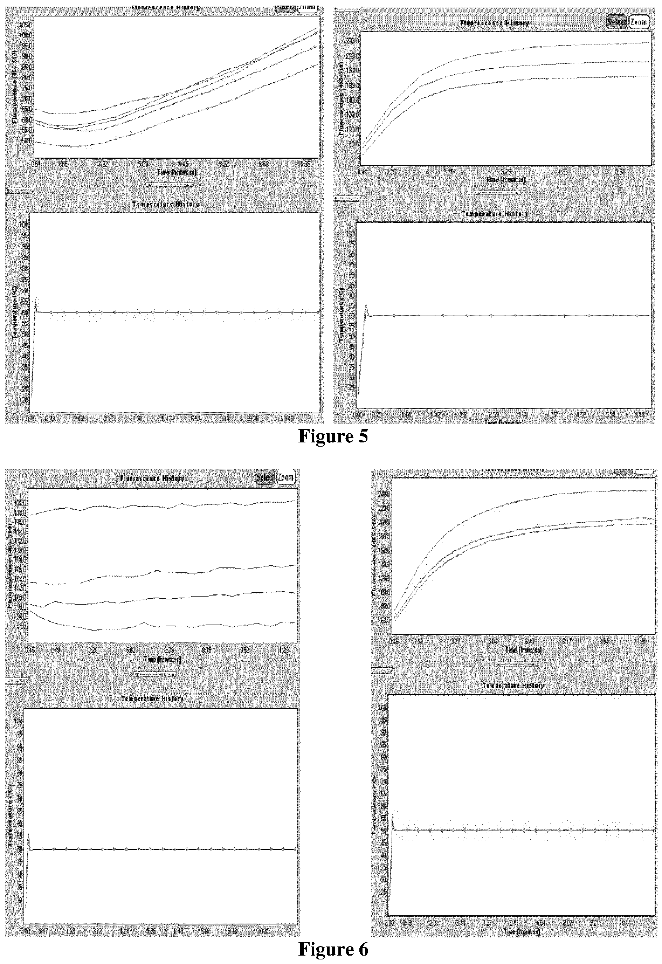

Figure 5 shows the results of isothermal extension amplification at 60°C; wherein the left graph shows the case where the nucleic acid ligand (nucleic acid polymerase substrate analog) is added (the upper shows an amplification curve and the lower shows a reaction temperature), and the right graph shows the case where no nucleic acid ligand is added (the upper shows an amplification curve and the lower shows a reaction temperature);
Figure 6 shows the results of isothermal extension amplification at 50°C; wherein the left graph shows the case where the nucleic acid ligand (nucleic acid polymerase substrate analog) is added (the upper shows an amplification curve and the lower shows a reaction temperature), and the right graph shows the case where no nucleic acid ligand (nucleic acid polymerase substrate analog) is added (the upper shows an amplification curve and the lower shows a reaction temperature);

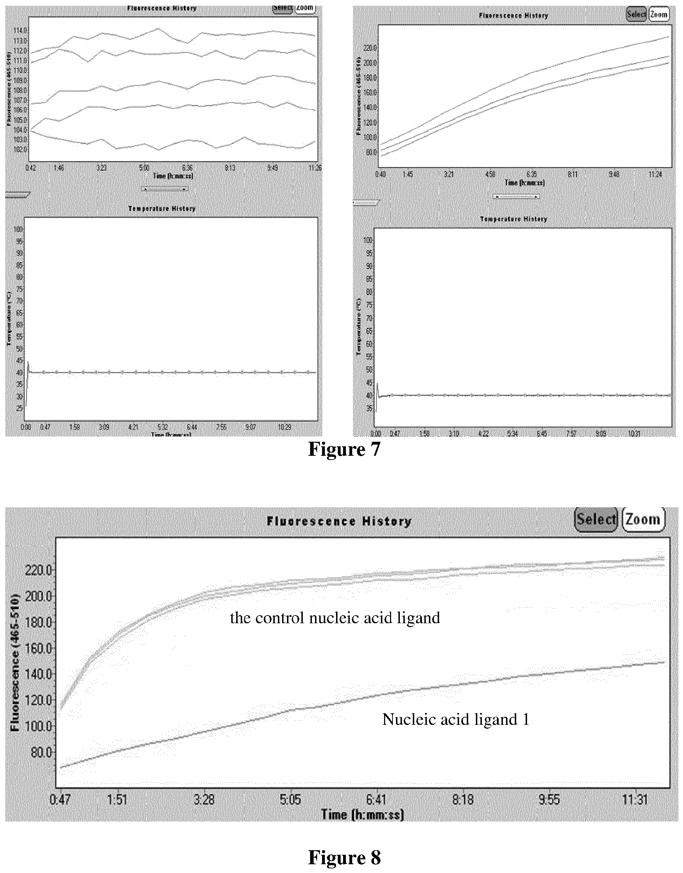

Figure 7 shows the results of isothermal extension amplification at 40°C; wherein the left graph shows the case where the nucleic acid ligand (nucleic acid polymerase substrate analog) is added (the upper shows an amplification curve and the lower shows a reaction temperature), and the right graph shows the case where no nucleic acid ligand (nucleic acid polymerase substrate analog) is added (the upper shows an amplification curve and the lower shows a reaction temperature);
Figure 8 shows the comparison result of amplification using a nucleic acid ligand (nucleic acid polymerase substrate analog) with and without 3'-end modification;

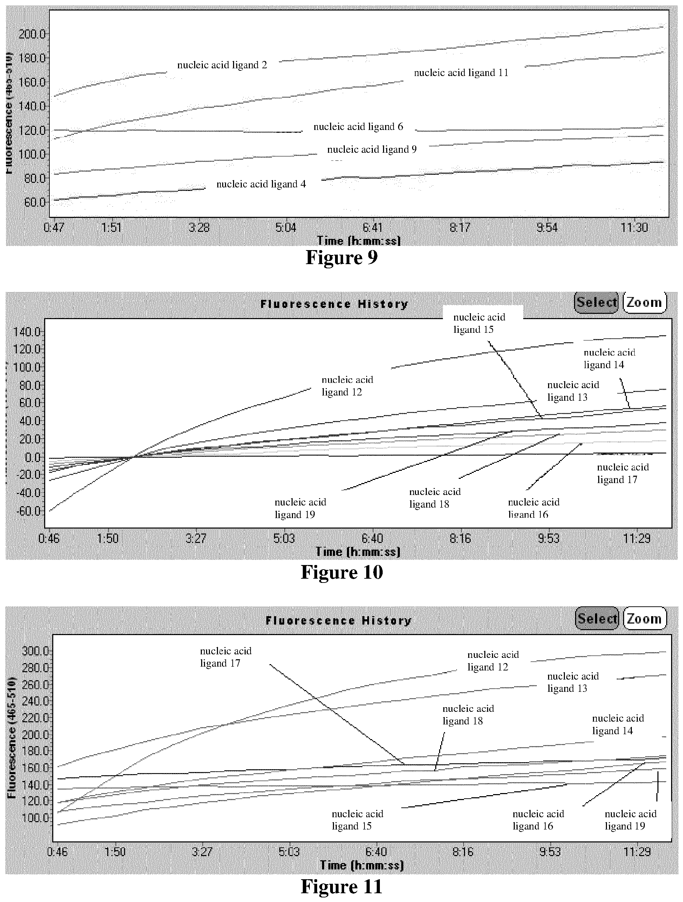

Figure 9 shows the inhibitory effect of the nucleic acid ligand (nucleic acid polymerase substrate analog) of the present invention (having different numbers of intramolecular complementary base pairing/different temperature stability) on enzyme activity;
Figure 10 shows the inhibitory effect of the nucleic acid ligand (nucleic acid polymerase substrate analog) of the present invention (intermolecular complementary pairing) on the enzyme activity of Taq enzyme;
Figure 11 shows the inhibitory effect of the nucleic acid ligand (nucleic acid polymerase substrate analog) of the invention (intermolecular complementary pairing) on the enzyme activity of KOD DNA polymerase;

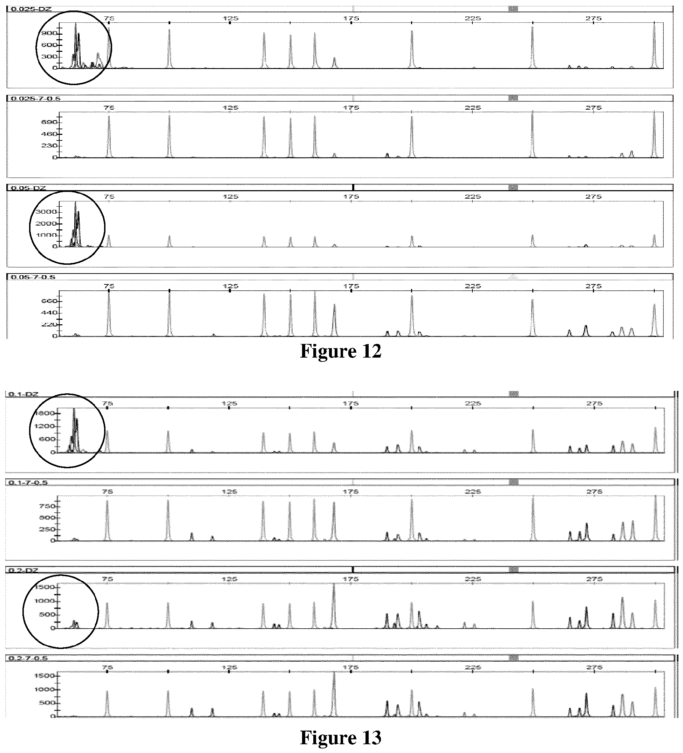

Figure 12 shows the amplification results of human genome 9948 with template amounts of 0.025ng and 0.05ng, wherein, from top to bottom, successively: the amplification result with a template amount of 0.025ng, without the addition of a nucleic acid ligand (a nucleic acid polymerase substrate analog); the amplification result with a template amount of 0.025ng, with the addition of a nucleic acid ligand (a nucleic acid polymerase substrate analog); the amplification result with a template amount of 0.05ng, without the addition of a nucleic acid ligand (a nucleic acid polymerase substrate analog); the amplification result with a template amount of 0.05ng, with the addition of a nucleic acid ligand (a nucleic acid polymerase substrate analog);
Figure 13 shows the amplification results of human genome 9948 with template amounts of 0.1ng and 0.2ng, wherein, from top to bottom, successively: the amplification result with a template amount of 0.1ng, without the addition of a nucleic acid ligand (a nucleic acid polymerase substrate analog); the amplification result with a template amount of 0.1ng, with the addition of a nucleic acid ligand (a nucleic acid polymerase substrate analog); the amplification result with a template amount of 0.2ng, without the addition of a nucleic acid ligand (a nucleic acid polymerase substrate analog); the amplification result with a template amount of 0.2ng, with the addition of a nucleic acid ligand (a nucleic acid polymerase substrate analog).

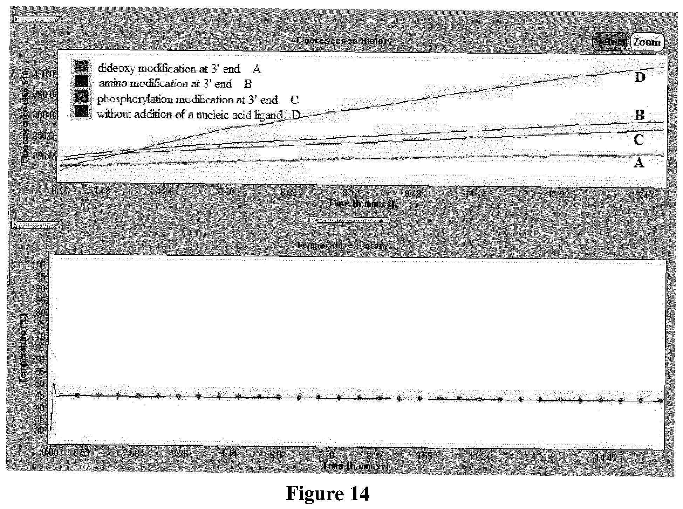

Figure 14 shows a comparison between nucleic acid ligands with different modifications at 3' end.

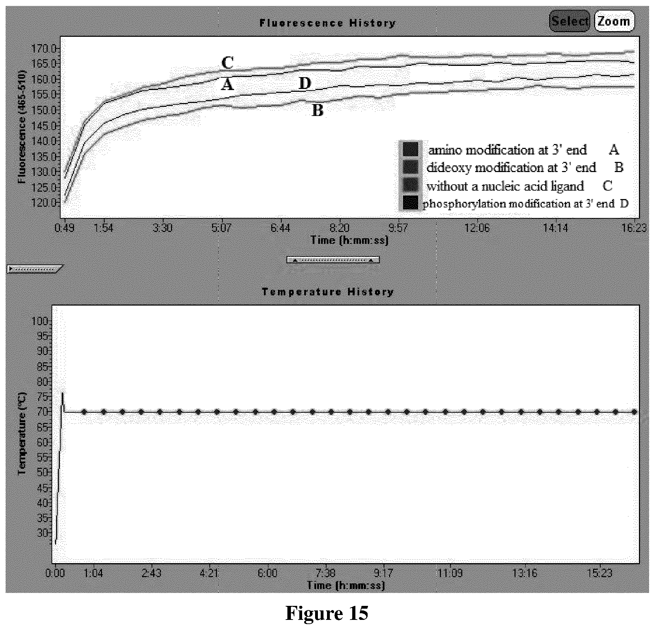

Figure 15 shows a comparison between nucleic acid ligands with different modifications at 3' end.

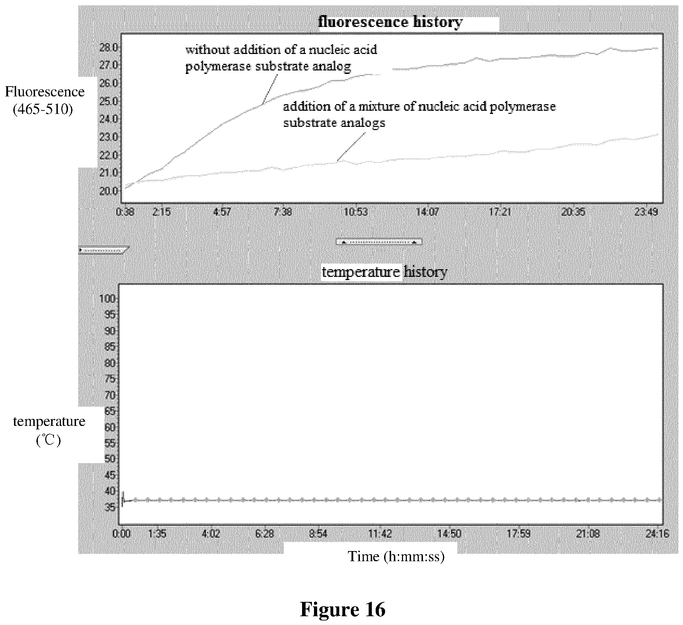

Figure 16 shows the enzyme activity of Reverse transcriptase without the addition of a nucleic acid polymerase substrate analog and with the addition of two nucleic acid polymerase substrate analogs during isothermal extension at 37°C; the upper graph is the curves of the enzyme activity without the addition of a nucleic acid polymerase substrate analog and with the addition of two nucleic acid polymerase substrate analogs, and the lower graph is the curve of the reaction temperature;

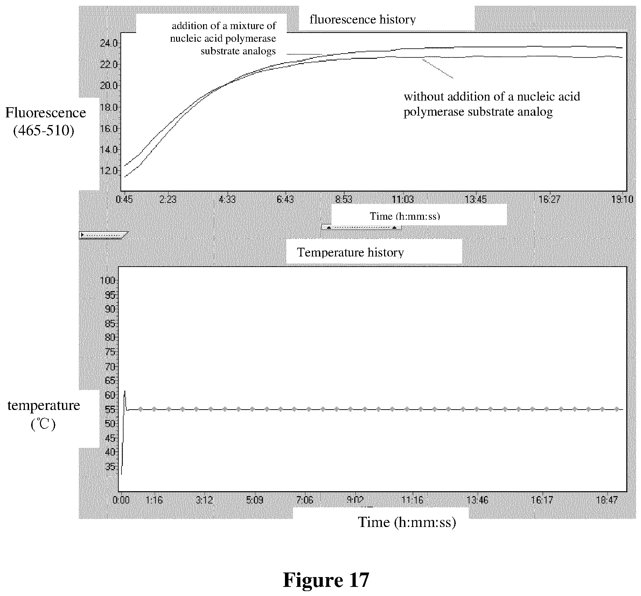

Figure 17 shows the enzyme activity of Reverse transcriptase without the addition of a nucleic acid polymerase substrate analog and with the addition of two nucleic acid polymerase substrate analogs during isothermal extension at 55°C; the upper graph is the curves of the enzyme activity without the addition of a nucleic acid polymerase substrate analog and with the addition of two nucleic acid polymerase substrate analogs, and the lower graph is the curve of the reaction temperature;

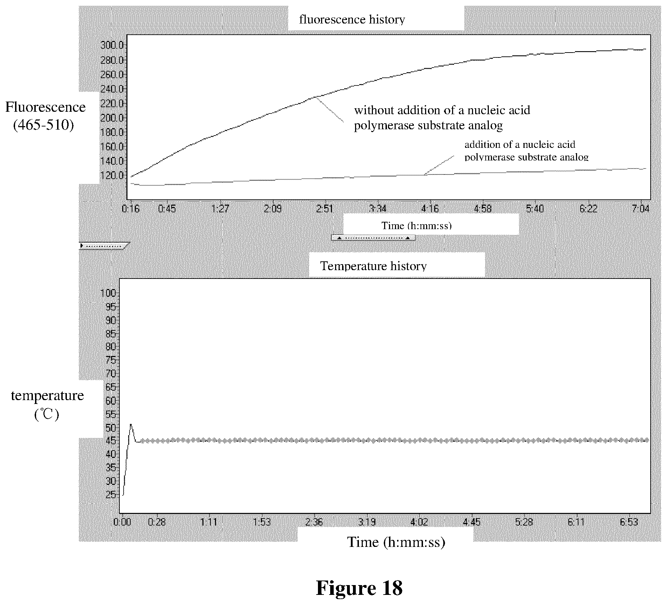

Figure 18 shows the enzyme activity of a DNA polymerase (BST DNA Polymerase) without the addition of a nucleic acid polymerase substrate analog and with the addition of one nucleic acid polymerase substrate analog during isothermal extension at 45°C; the upper graph is the curves of the enzyme activity without the addition of a nucleic acid polymerase substrate analog and with the addition of one nucleic acid polymerase substrate analog, and the lower graph is the curve of the reaction temperature;

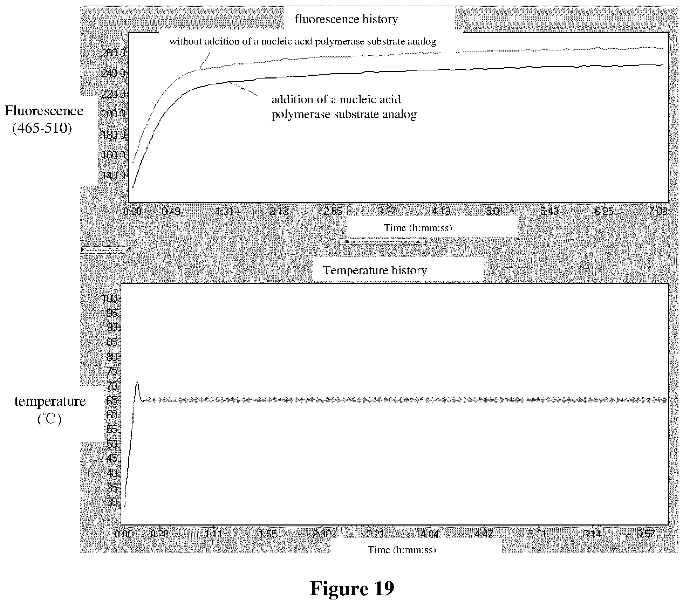

Figure 19 shows the enzyme activity of a DNA polymerase (BST DNA Polymerase) without the addition of a nucleic acid polymerase substrate analog and with the addition of one nucleic acid polymerase substrate analog during isothermal extension at 65°C; the upper graph is the curves of the enzyme activity without the addition of a nucleic acid polymerase substrate analog and with the addition of one nucleic acid polymerase substrate analog, and the lower graph is the curve of the reaction temperature;

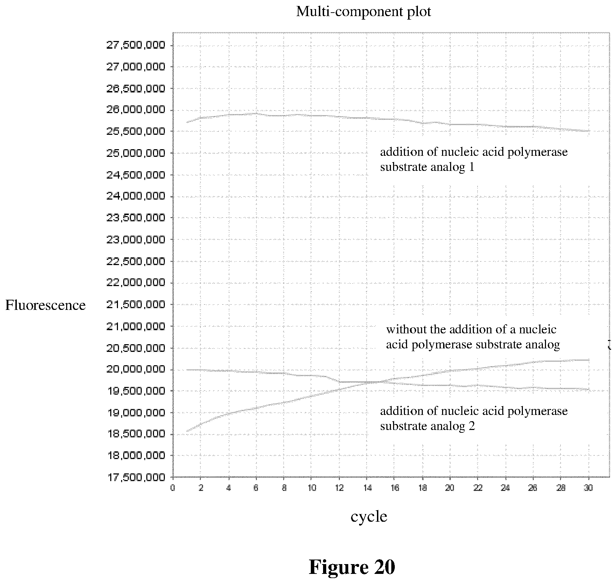

Figure 20 shows the enzyme activity of TAQ enzyme without the addition of a nucleic acid polymerase substrate analog and with the addition of the nucleic acid polymerase substrate analogs 1 and 2 respectively during isothermal extension at 65°C;

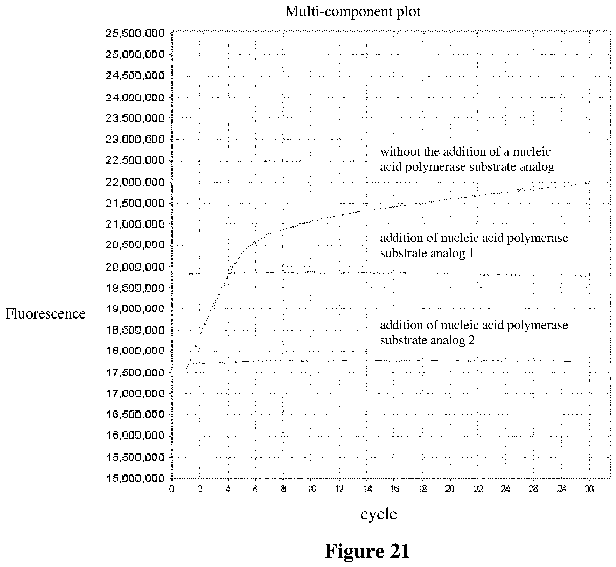

Figure 21 shows the enzyme activity of TAQ enzyme without the addition of a nucleic acid polymerase substrate analog and with the addition of the nucleic acid polymerase substrate analogs 1 and 2 respectively during isothermal extension at 40°C;

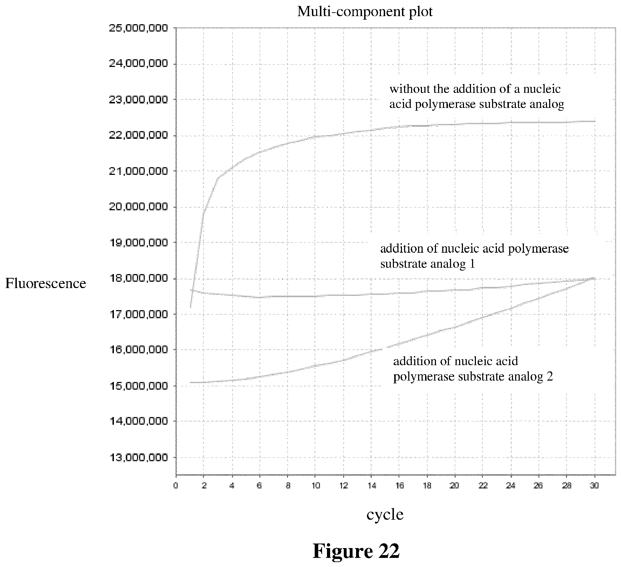

Figure 22 shows the enzyme activity of TAQ enzyme without the addition of a nucleic acid polymerase substrate analog and with the addition of the nucleic acid polymerase substrate analogs 1 and 2 respectively during isothermal extension at 50°C;

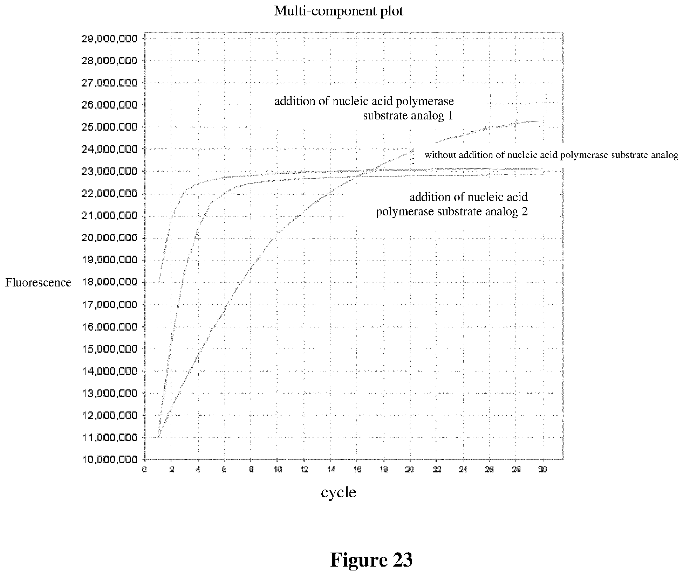

Figure 23 shows the enzyme activity of TAQ enzyme without the addition of a nucleic acid polymerase substrate analog and with the addition of the nucleic acid polymerase substrate analogs 1 and 2 respectively during isothermal extension at 60°C;

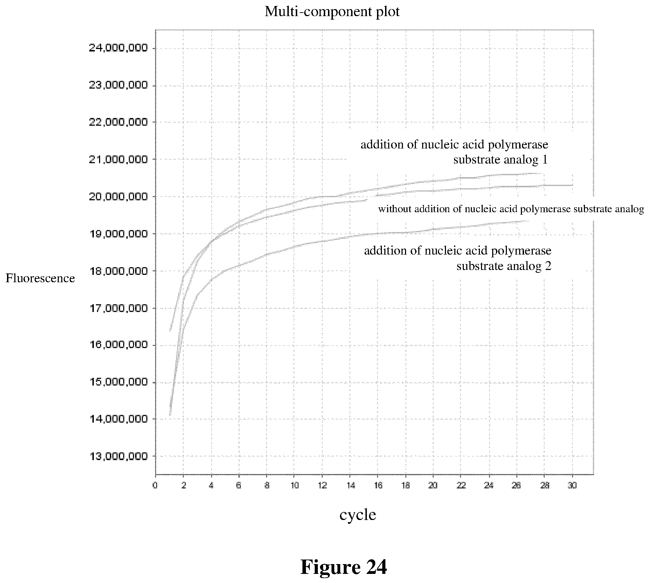

Figure 24 shows the enzyme activity of TAQ enzyme without the addition of a nucleic acid polymerase substrate analog and with the addition of the nucleic acid polymerase substrate analogs 1 and 2 respectively during isothermal extension at 70°C;

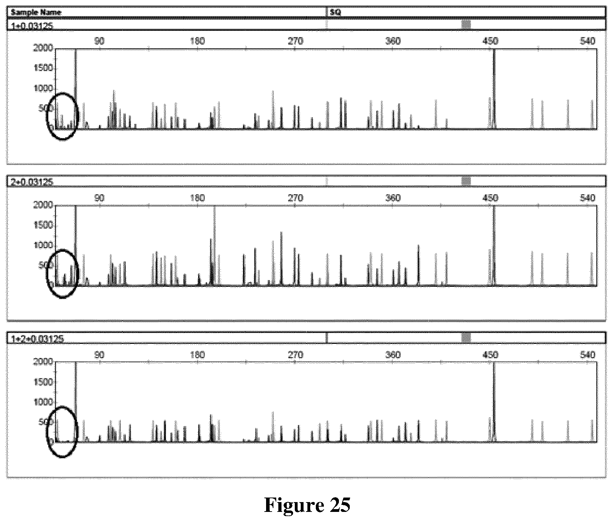

Figure 25 shows the amplification results of human genome M2 with a template amount of 0.03125ng, wherein, from top to bottom, successively: the amplification result with a template amount of 0.03125ng, with the TAQ enzyme modified by the nucleic acid polymerase substrate analog 1; the amplification result with a template amount of 0.03125ng, with the TAQ enzyme modified by the nucleic acid polymerase substrate analog 2; the amplification result with a template amount of 0.03125ng, with the TAQ enzyme modified by the nucleic acid polymerase substrate analog 1 and the nucleic acid polymerase substrate analog 2 mixed in an equal ratio;

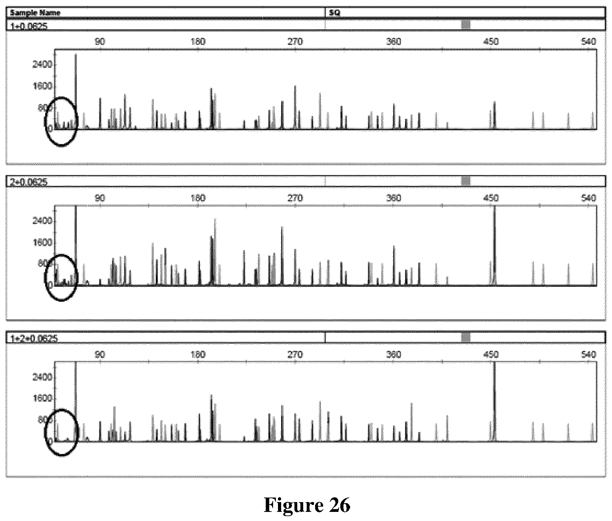

Figure 26 shows the amplification results of human genome M2 with a template amount of 0.0625ng, wherein, from top to bottom, successively: the amplification result with a template amount of 0.0625ng, with the TAQ enzyme modified by the nucleic acid polymerase substrate analog 1; the amplification result with a template amount of 0.0625ng, with the TAQ enzyme modified by the nucleic acid polymerase substrate analog 2; the amplification result with a template amount of 0.0625ng, with the TAQ enzyme modified by the nucleic acid polymerase substrate analog 1 and the nucleic acid polymerase substrate analog 2 mixed in an equal ratio;

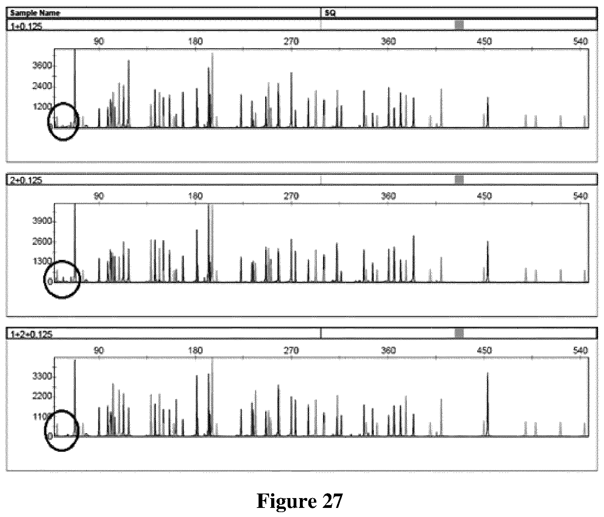

Figure 27 shows the amplification results of human genome M2 with a template amount of 0.125ng, wherein, from top to bottom, successively: the amplification result with a template amount of 0.125ng, with the TAQ enzyme modified by the nucleic acid polymerase substrate analog 1; the amplification result with a template amount of 0.125ng, with the TAQ enzyme modified by the nucleic acid polymerase substrate analog 2; the amplification result with a template amount of 0.125ng, with the TAQ enzyme modified by the nucleic acid polymerase substrate analog 1 and the nucleic acid polymerase substrate analog 2 mixed in an equal ratio;

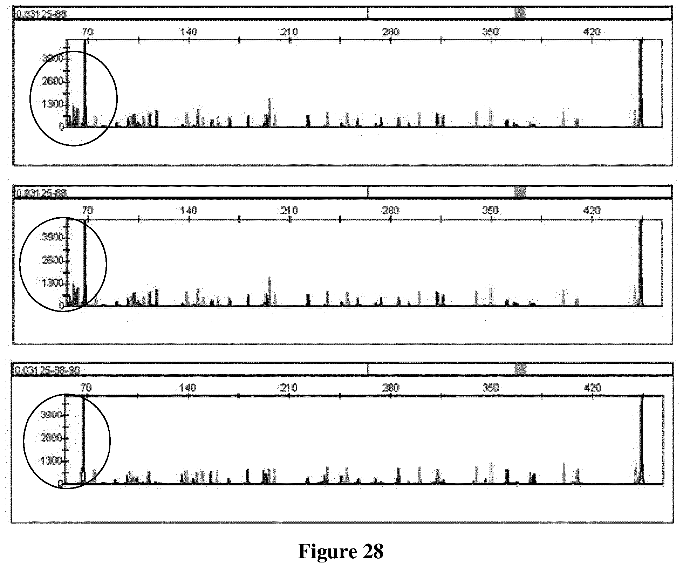

Figure 28 shows the amplification results of the human genome gene with a template amount of 0.03125ng, with the addition of different nucleic acid polymerase substrate analogs and being left at 4°C for 1 day, wherein, from top to bottom, successively: the amplification result with the addition of the nucleic acid polymerase substrate analog 1, the amplification result with the addition of a mixture of the nucleic acid polymerase substrate analogs 1 and 2, and the amplification result with the addition of a mixture of the nucleic acid polymerase substrate analogs 1, 2 and 3;

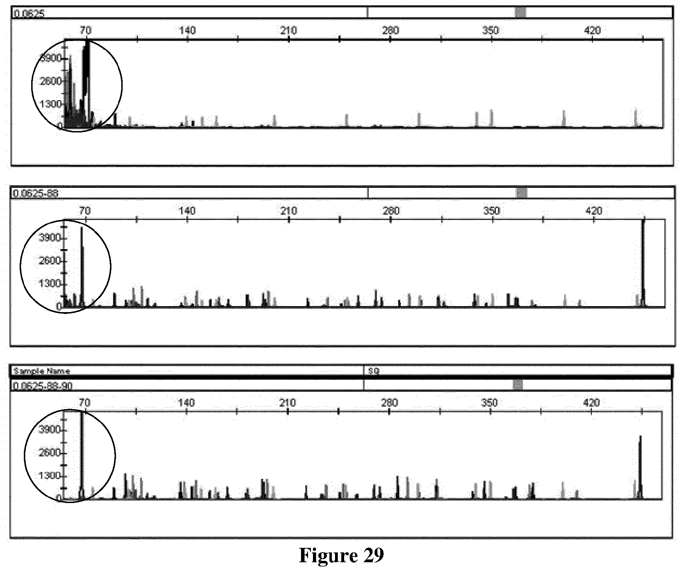

Figure 29 shows the amplification results of the human genome gene with a template amount of 0.0625ng, with the addition of different nucleic acid polymerase substrate analogs and being left at 4°C for 1 day, wherein, from top to bottom, successively: the amplification result with the addition of the nucleic acid polymerase substrate analog 1, the amplification result with the addition of a mixture of the nucleic acid polymerase substrate analogs 1 and 2, and the amplification result with the addition of a mixture of the nucleic acid polymerase substrate analogs 1, 2 and 3;

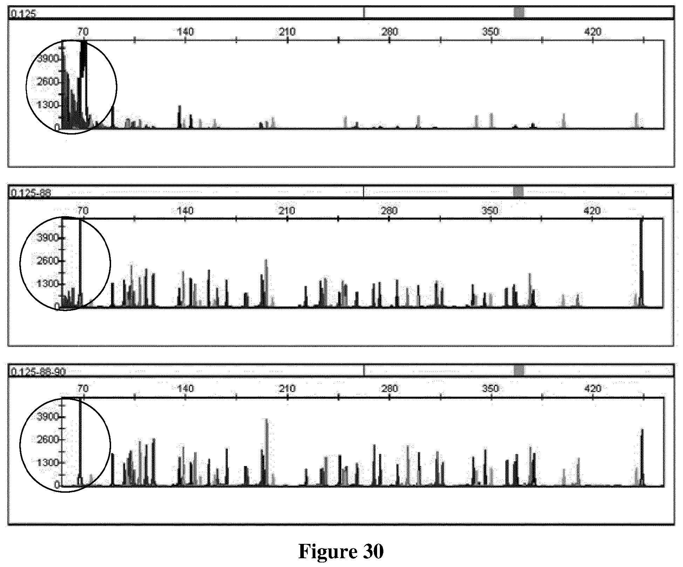

Figure 30 shows the amplification results of the human genome gene with a template amount of 0.125ng, with the addition of different nucleic acid polymerase substrate analogs and being left at 4°C for 1 day, wherein, from top to bottom, successively: the amplification result with the addition of the nucleic acid polymerase substrate analog 1, the amplification result with the addition of a mixture of the nucleic acid polymerase substrate analogs 1 and 2, and the amplification result with the addition of a mixture of the nucleic acid polymerase substrate analogs 1, 2 and 3;

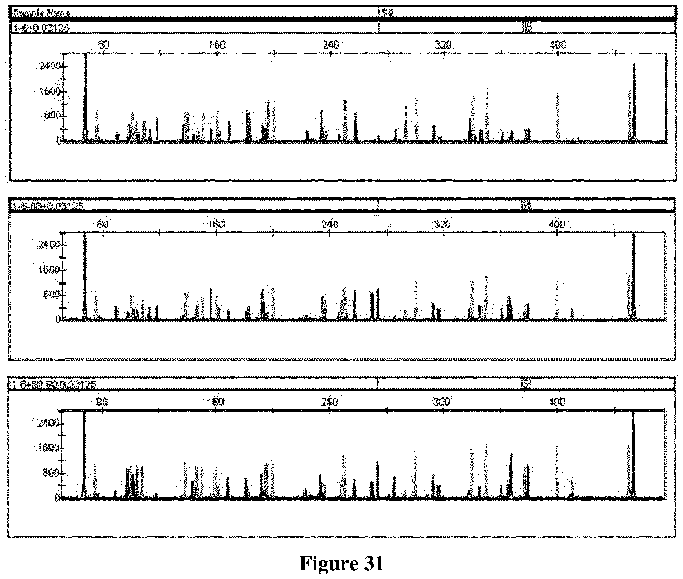

Figure 31 shows the direct amplification results of the human genome gene with a template amount of 0.03125ng, with the addition of different nucleic acid polymerase substrate analogs and without placement, wherein, from top to bottom, successively: the amplification result with the addition of the nucleic acid polymerase substrate analog 1, the amplification result with the addition of a mixture of the nucleic acid polymerase substrate analogs 1 and 2, and the amplification result with the addition of a mixture of the nucleic acid polymerase substrate analogs 1, 2 and 3;

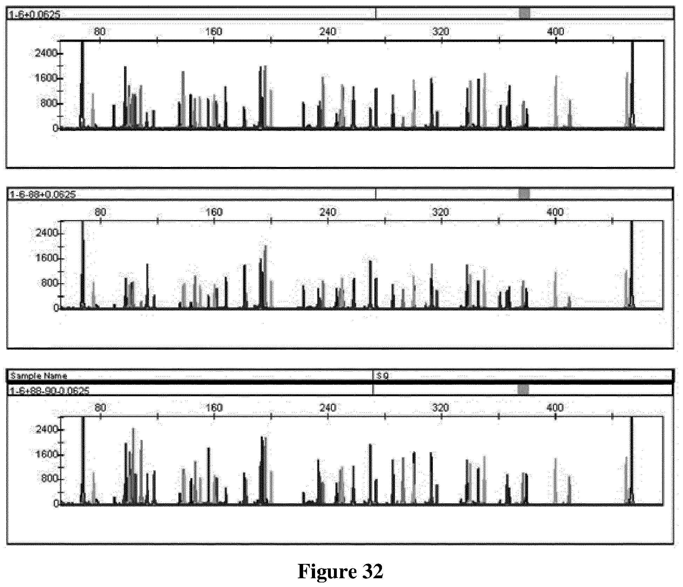

Figure 32 shows the direct amplification results of the human genome gene with a template amount of 0.0625ng, with the addition of different nucleic acid polymerase substrate analogs and without placement, wherein, from top to bottom, successively: the amplification result with the addition of the nucleic acid polymerase substrate analog 1, the amplification result with the addition of a mixture of the nucleic acid polymerase substrate analogs 1 and 2, and the amplification result with the addition of a mixture of the nucleic acid polymerase substrate analogs 1, 2 and 3;

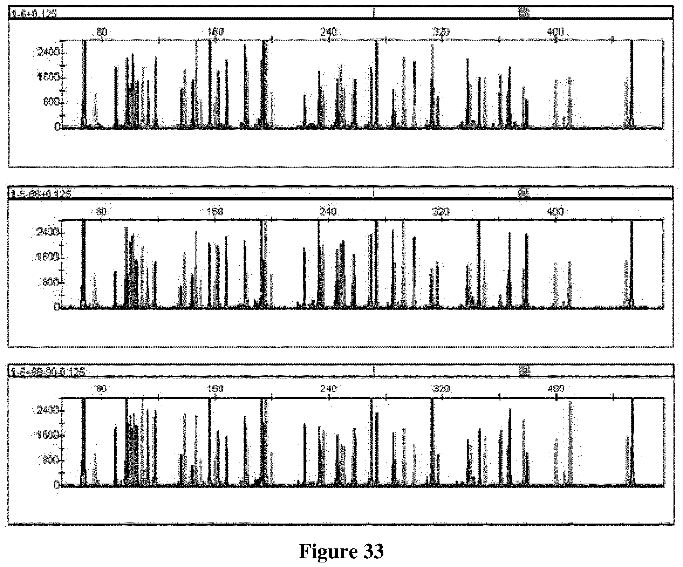

Figure 33 shows the direct amplification results of the human genome gene with a template amount of 0.125ng, with the addition of different nucleic acid polymerase substrate analogs and without placement, wherein, from top to bottom, successively: the amplification result with the addition of the nucleic acid polymerase substrate analog 1, the amplification result with the addition of a mixture of the nucleic acid polymerase substrate analogs 1 and 2, and the amplification result with the addition of a mixture of the nucleic acid polymerase substrate analogs 1, 2 and 3;

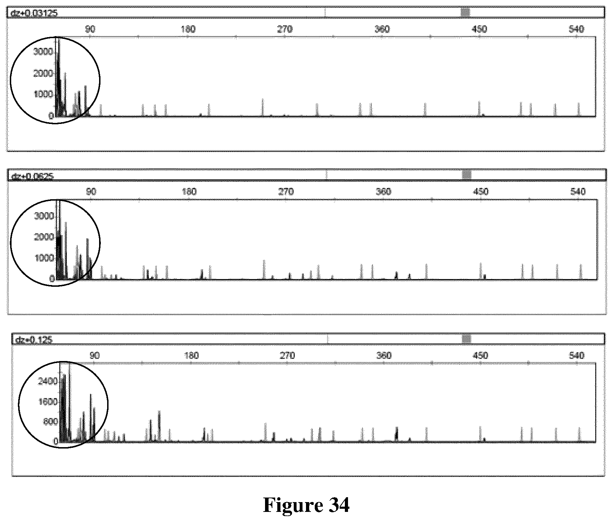

Figure 34 shows the amplification results of the human genome gene with different template amounts, without the addition of a nucleic acid polymerase substrate analog and being left at 4°C for 1 day, wherein, from top to bottom: the amplification results of the human genome gene with a template amount of 0.03125ng, 0.0625ng and 0.125ng, successively;

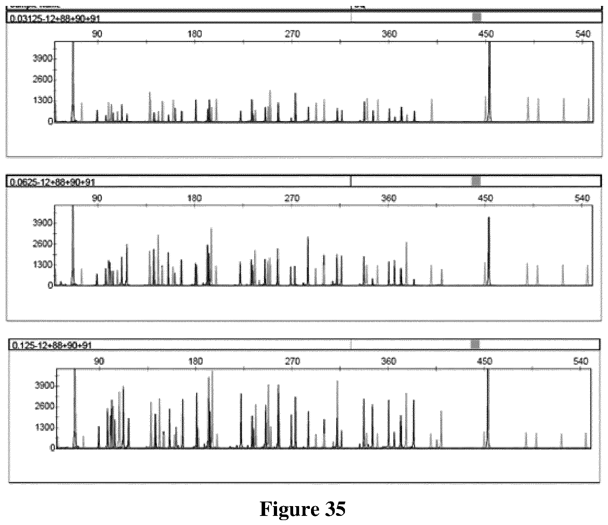

Figure 35 shows the amplification results of the human genome gene with different template amounts, with the addition of a mixture of the nucleic acid polymerase substrate analogs 1, 2, 3, and 4, and being left at 4°C for 1 day, wherein, from top to bottom: the amplification results of the human genome gene with a template amount of 0.03125ng, 0.0625ng and 0.125ng, successively;

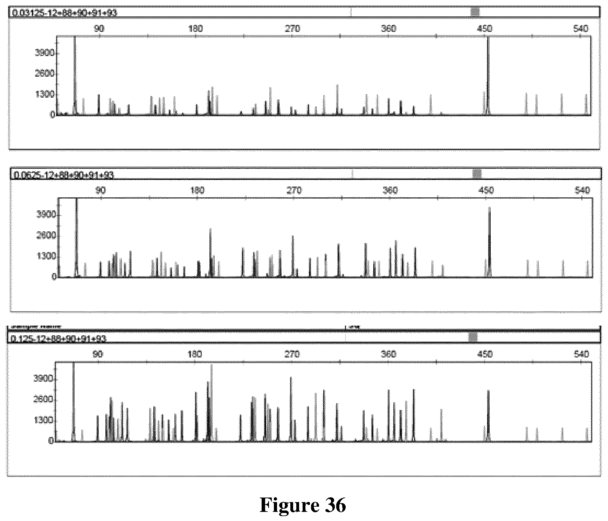

Figure 36 shows the amplification results of the human genome gene with different template amounts, with the addition of a mixture of the nucleic acid polymerase substrate analogs 1, 2, 3, 4 and 5, and being left at 4°C for 1 day, wherein, from top to bottom: the amplification results of the human genome gene with a template amount of 0.03125ng, 0.0625ng and 0.125ng, successively.

### DETAILED DESCRIPTION OF THE INVENTION

The present invention discloses a nucleic acid ligand (nucleic acid polymerase substrate analog), or a mixture thereof, and use thereof, and those skilled in the art can learn from the content of this article and appropriately improve the process parameters to achieve. It should be particularly pointed out that all similar substitutions and modifications are obvious to those skilled in the art, and they are deemed to be included in the present invention. The nucleic acid ligand (nucleic acid polymerase substrate analog), or a mixture thereof, and use thereof according to the present invention have been described through the preferred examples, and relevant persons can obviously change or suitably modify and combine the nucleic acid ligand (nucleic acid polymerase substrate analog) and use thereof described herein without departing from the content, spirit and scope of the present invention, to realize and apply the technology of the present invention.

In the specific embodiments of the present invention, the raw materials used in each treatment group of the provided comparative test are the same, and the other test conditions of each group are kept the same except the due differences. The raw materials, reagents, etc. involved in the present invention can be obtained through commercially available channels unless otherwise specified.

Unless particularly defined, all the scientific or technological terminologies in the present invention are the same as general understandings thereof to most of general persons in the art. Most of the terminologies in the art have general meanings in the following documents, all professional terms in the present invention are the same as these described in the above documents.

The term "nucleotide" generally refers to a compound which is formed from a nucleoside linked to an acidic molecule or group via an ester bond, for example, nucleoside phosphate, which commonly has one, two or three phosphate groups covalently linked at position 5 of the glycosyl group in the nucleoside. In some cases, the definition of nucleotide also involves homologues or analogs of some typical nucleotides. 2' deoxynucleotide triphosphate is typically used by DNA polymerases to synthesize DNA.

The term "nucleic acid" includes deoxyribonucleic acids (DNAs), ribonucleic acids (RNAs), DNA-RNA hybrids, oligonucleotides, aptamers, peptide nucleic acids (PNAs), PNA-DNA hybrids, PNA-RNA hybrids, and the like. All covalently linked nucleotides in a linear (single-stranded or double-stranded) or branched form are comprised. A typical nucleic acid is generally single-stranded or double-stranded, and comprises a phosphodiester bond.

The term "amplification" refers to a process that the number of a target nucleic acid fragment is increased under the action of a nucleic acid polymerase, which includes but is not limited to a polymerase chain reaction (PCR), a ligase chain reaction (LCR), nucleic acid sequence-based amplification (NASBA)), etc.

In examples of the present invention, "amplification" refers to a polymerase chain reaction (PCR). After denaturation and dissociation of a template, an oligonucleotide primer is annealed and hybridized with the template, accompanied by addition of nucleotides and strand extension. This is repeated for a certain cycles to achieve the amplification of a target nucleotide fragment.

The term "thermophilic enzyme" refers to an enzyme that is stable to heat and promotes the polymerization of nucleotides to form polynucleotide extension products. Typically, thermophilic and stable polymerases are commonly used during thermal cycling. During PCR cycling, double-stranded nucleotides are denatured by high temperatures (e.g. 95°C). The thermophilic enzymes described herein that are effective for use in PCR amplification reactions meet at least one criterion, i.e., the enzyme is not denatured when subjected to an elevated temperature for a period necessary to achieve denaturation of double-stranded nucleotides. In some experimental systems, the thermophilic enzymes will not be denatured from 90°C to 100 °C.

As used herein, a "nucleic acid ligand" (a nucleic acid polymerase substrate analog) is a non-naturally occurring nucleic acid that has a desired effect on a nucleic acid polymerase.

A "nucleic acid polymerase substrate analog" is a non-naturally substance that can non-covalently binds to a nucleic acid polymerase and consists of oligomeric nucleic acids. In a preferred embodiment, the nucleic acid polymerase substrate analog has a binding affinity to a nucleic acid polymerase molecule, wherein the nucleic acid polymerase substrate analog is not a nucleic acid having a known physiological function of binding to a target molecule.

The nucleic acid ligands (nucleic acid polymerase substrate analogs) used herein bind to a nucleic acid polymerase by mimicking a substrate of the nucleic acid polymerase, and are a single or two nucleic acid molecules or nucleic acid molecule analogs capable of forming intramolecular or intermolecular complementary pairing, wherein these nucleic acid molecules or nucleic acid molecule analogs are modified at 3' end so as to stop the extension of the polymerase, and stable at or below a certain temperature; the paired nucleotides dissociate into a single strand in a heating state, and the nucleic acid polymerase detaches from the nucleic acid ligand (nucleic acid polymerase substrate analog) to exert its intrinsic function.

"Nucleic acid" refers to DNA, RNA, which is single or double stranded and may have any chemical modification. Modifications include, but are not limited to, those that provide other chemical groups that incorporate additional charge, polarizability, hydrogen bonding, electrostatic interactions, and fluxes to the nucleic acid ligand base or the entire nucleic acid ligand. Such modifications include, but are not limited to, 2'-position sugar modification, 5-position pyrimidine modification, 8-position purine modification, modifications on exocyclic amines, substitution of 4-thiouridine, substitution of 5-bromo or 5-iodo-. Uracil, modifications on a backbone chain, methylation, unusual base-pairing combinations such as isobases, isocytidine and isoguanidine, and the like. Modifications may also include 3' and 5' modifications, such as capping.

The method of "enzyme activity assay by isothermal extension" involves the performance evaluation of the selected nucleic acid ligands (nucleic acid polymerase substrate analogs), which interact with a polymerase in an ideal manner. In the present invention, the nucleic acid ligand (nucleic acid polymerase substrate analog) for a nucleic acid polymerase is verified using the method of enzyme activity assay by isothermal extension.

As used herein, "for a nucleic acid ligand (nucleic acid polymerase substrate analog)" is a nucleic acid ligand (nucleic acid polymerase substrate analog) identified by an isothermal extension method, which modulates the affinity to its taq enzyme based on temperature stability parameters. In preferred embodiments, the primary parameter is temperature, and the affinity of the nucleic acid ligand (nucleic acid polymerase substrate analog) to its taq enzyme decreases at an elevated temperature.

As used herein, "nucleic acid polymerase" refers to any enzyme that catalyzes the synthesis of DNA by using DNA or RNA (reverse transcriptase) as a template and adding deoxyribonucleotide units to a DNA strand.

A "switch" refers to any compound that acts to start or shut down a reaction depending on some specific reaction conditions. In the present invention, the function of the nucleic acid ligand (nucleic acid polymerase substrate analogue) is to "switch on" or "switch off" the PCR according to the following conditions.

The 3' end of the nucleic acid polymerase substrate analogs of the invention has modifications that inhibit their extension, including but not limited to dideoxy modification, phosphorylation modification or amino modification and the like. The dideoxy modification, phosphorylation modification or amino modification can be carried out using methods known in the art. For example, the dideoxy modification can mix primers with any one of four dideoxynucleotides (ddATP, ddTTP, ddCTP, or ddGTP) by utilizing the property of a terminal transferase (TdT) to catalyze the binding of deoxynucleotides (dNTPs) or dideoxynucleotides (ddNTPs) to the 3' hydroxyl terminus of a DNA molecule. The TdT can add dideoxynucleotides to the 3' end of primers, and the resulting ddNTP-modified primers cannot be extended by DNA polymerase. Invitrogen provides amino modifications at 3' end (AminolinkerC6/7/12). Phosphorylation at 3' end is conventionally achieved using phosphate-ON (also known as chemical phosphorylation reagent (CPR)), for example incorporation of a 3' phosphate by addition to any support (e.g. dT column). 3'-Phosphorylation is used to block enzyme activity.

In specific embodiments of the invention, the invention provides a variety of nucleic acid ligands (nucleic acid polymerase substrate analogs), wherein the sequences of the nucleic acid ligands with intramolecular complementary pairing are shown in SEQ ID NO: 1-11, the sequence of one strand of the nucleic acid ligands with intermolecular complementary pairing is shown in SEQ ID NO: 12, and the sequence of the other strand is shown in SEQ ID NO: 13-20. In specific embodiments of the present invention, Taq polymerase and KOD polymerase are used mainly for illustration, but in practice the nucleic acid ligands (nucleic acid polymerase substrate analogs) described herein are suitable for all nucleic acid polymerases and nucleic acid amplification reactions.

In other specific embodiments of the invention, the invention provides a variety of nucleic acid polymerase substrate analogs as shown in SEQ ID NO: 21-28. In specific embodiments of the present invention, reverse transcriptase, BST DNA polymerase and TAQ enzyme are used mainly for illustration, but in practice the nucleic acid polymerase substrate analogs described herein are suitable for all nucleic acid polymerases and nucleic acid amplification reactions.

The technical solutions provided by the present invention will be further described through the following examples. The following examples are merely used to demonstrate the invention, but not to limit the scope of the invention.

### Example 1: Assay for inhibition of polymerase activity by nucleic acid ligands (nucleic acid polymerase substrate analogs)

The enzyme activity is determined by a single-chain extension method using commercially available NEB M13 single-chain DNA and related primers. A real-time detection is performed by fluorescent quantitative method using an instrument of Roche LC480II.

The sequence of Primer M13R and the amplification system are set forth as follows:
M13R primer 1 ACGCTCGTCATCAAAATCACTCGCATCAACCAAACCGTTAT

**Table 1**

| Component | Concentration | | Volume added per aliquot µl | |
|---|---|---|---|---|
| 10x Buffer A | | 10 x | | 2.5 |
| MgCl₂ | | 250mM | | 0.5 |
| SG | | 100 x | | 0.4 |
| M13ssDNA | | 0.73mg/ml | | 0.45 |
| dNTP | | 100mM | | 0.2 |
| M13R | | 100µM | | 0.1 |
| taq enzyme | | / | | 1 |
| ddH₂O | | / | | 19.85 |

Wherein, 10X bufferA is a buffer prepared from 30mM, Tris 8.0; 50mM KCl; TWEEN20 0.05%; 10mM mercaptoethanol. This reaction system has high repeatability of accuracy in an range of 0.04-0.008U for the original enzyme.

The reaction program for the isothermal extension reaction is: (72°C, 30s) * 22 cycles, and the reaction system was set as 25µl.

The polymerase was diluted from 0.04U to 0.008U as follows:

**Table 2**

| Name | Enzyme amount U | | volume of mother liquor µl | | volume of 1x bufferA µl | |
|---|---|---|---|---|---|---|
| Control enzyme | | 0.04 | | 4 | | 96 |
| | | 0.024 | | 12 | | 8 |
| | | 0.02 | | 10 | | 10 |
| | | 0.016 | | 8 | | 12 |
| | | 0.012 | | 6 | | 14 |
| | | 0.008 | | 4 | | 16 |
| | | 0.004 | | 2 | | 18 |

As shown in the results of Figure 3, R²>0.99, this conforms to the linear theory. This reaction can be sensitive enough to detect the blocking effect on DNA polymerase in different temperature ranges.

### Example 2: Polymerase blocking experiment (selection of optimal isothermal extension conditions for screening nucleic acid ligands)

The extension at 70°C, 60°C, 50°C, 40°C is performed respectively according to the method of Example 1 to screen which nucleic acid ligands could achieve the desired effect, for example the following nucleic acid ligand:
Nucleic acid ligand (nucleic acid polymerase substrate analog) 1: TCGAACGGTATATATATTAATATATATATAC (as shown in SEQ ID NO: 1), with dideoxy modification at 3' end.

6U of DNA enzyme was mixed with the above nucleic acid ligand 1, and then mixed according to addition of about 6U of DNA enzyme for 100uM 0.05ul system, and the mixture was tested at -20°C overnight. Meanwhile, the system without a nucleic acid ligand was used as the control system. According to the activity assay of Example 1, the enzyme activities of the two systems were tested respectively.

It can be seen from Figures 4-7 that in the system without a nucleic acid ligand, the signal of the polymerase increases significantly at 40°C and 50°C, while in the system with a nucleic acid ligand, the activity of the polymerase is partially inhibited, and in particular, is completely lost below 50°C. In addition, the amount of 6U of the original enzyme added is generally large in a PCR experiment, and the added amount in an actual PCR experiment is far less than 6U. When the temperature is below 50°C, this system can completely block the activity of the polymerase to allow it to lose the residual enzyme activity, thereby achieving the effect of inhibiting the non-specific amplification.

Therefore, it was finally confirmed that the following examples were experimentally tested at 50°C to screen the test nucleic acid ligands.

### Example 3: Polymerase blocking experiment

The modification at 3' end of the nucleic acid molecules or nucleic acid molecule analogs may take the conventional form for amplification prevention (such as dideoxy modification, phosphorylation modification, amino modification, etc.), and the dideoxy method is selected for terminating the terminal extension in this example. The nucleic acid molecule unmodified at 3' end was also used as a control to test whether an unmodified nucleic acid molecule could also inhibit enzyme activity. The experimental method may refer to Example 1. The sequences of the two nucleic acid ligands (nucleic acid polymerase substrate analogs) are set forth as follows:
Nucleic acid ligand (Nucleic acid polymerase substrate analog) 1: TCGAACGGTATATATATTAATATATATATAC (as shown in SEQ ID NO: 1), with dideoxy modification at 3' end;
Control nucleic acid ligand (Nucleic acid polymerase substrate analog): TCGAACGGTATATATATTAATATATATATAC, unmodified at 3' end.

6U of DNA polymerase was mixed with the above nucleic acid molecule 2 and nucleic acid molecule 3 respectively, and experiments were performed using the resulting enzyme systems. For a 100uM 0.05ul system, about 6U of DNA enzyme was added. The mixture was tested at -20°C overnight.

The results are shown in Figure 8. The enzyme activity with the addition of the control nucleic acid ligand (nucleic acid polymerase substrate analog) remained the complete activity and increased rapidly; while in the system with the addition of the modified nucleic acid ligand (nucleic acid polymerase substrate analog) 1, the enzyme activity is mostly inhibited to achieve the desired result and the system can be blocked. This experiment demonstrates that 3' modifications of the nucleic acid ligands (nucleic acid polymerase substrate analogs) (not limiting to dideoxy modification, but including all 3' modifications that may prevent the DNA enzyme from extending) are important to the present invention.

### Example 4: Comparison of different modifications at 3' end of nucleic acid ligands (nucleic acid polymerase substrate analogs)

In this example, the nucleic acid ligand (nucleic acid polymerase substrate analog) 1 is modified at the last base at 3' end, with dideoxy modification, phosphorylation modification and amino modification respectively. It was tested whether the nucleic acid ligand with different modifications at 3' end could inhibit the enzyme activity during the isothermal extension at 45°C and 70 °C.
Nucleic acid ligand (nucleic acid polymerase substrate analog) 1: TCGAACGGTATATATATTAATATATATATAC (as shown in SEQ ID NO: 1), with dideoxy modification, phosphorylation modification and amino modification at 3' end;
Control nucleic acid ligand (nucleic acid polymerase substrate analog): TCGAACGGTATATATATTAATATATATATAC, unmodified at 3' end;

### Reaction system:

| Component | Volume added per aliquot ul |
|---|---|
| 10x bufferA | 2.5 |
| 1M MgCl₂ | 0.125 |
| 25mM each dNTPs | 0.2 |
| 100x SG | 0.4 |
| 100uM primer | 0.1 |
| 0.73mg/ml DNA | 0.45 |
| 5U/ul TAQ enzyme | 1 |
| 5uM nucleic acid ligand (nucleic acid polymerase substrate analog) | 1 |
| ddH2O | 19.225 |

The experimental result is shown in Figure 14. It can be seen from the experimental result that the enzyme activity with the addition of the control nucleic acid ligand (nucleic acid polymerase substrate analog) remained the complete activity and increases rapidly; while the addition of the nucleic acid ligand (nucleic acid polymerase substrate analog) with dideoxy modification or phosphorylation modification or amino modification at 3' end can effectively inhibit the activity of the polymerase during the isothermal extension at 45°C.

The experimental result is shown in Figure 15. It can be seen from the experimental result that the addition of the nucleic acid ligand (nucleic acid polymerase substrate analog) with dideoxy modification or phosphorylation modification or amino modification at 3' end could result in 100% release of polymerase activity during the isothermal extension at 70°C.

### Example 5: Screening of polymerase binding sequences (intramolecular)

The following nucleic acid ligands (nucleic acid polymerase substrate analogs) (their sequences are shown in SEQ ID NO: 2-11 successively) are modified at the last base at 3' end. The dideoxy modification at 3' end is used in this example.
Nucleic acid ligand (Nucleic acid polymerase substrate analog) 2: TCGAACGGGTATACC;
Nucleic acid ligand (Nucleic acid polymerase substrate analog) 3: TCGAACGGGATATATCC;
Nucleic acid ligand (Nucleic acid polymerase substrate analog) 4: TCGAACGGGATTATAATCC;
Nucleic acid ligand (Nucleic acid polymerase substrate analog) 5: TCGAACGGGATATATATATCC;
Nucleic acid ligand (Nucleic acid polymerase substrate analog) 6: TCGAACGGGATATACTATAGTATATCC;
Nucleic acid ligand (Nucleic acid polymerase substrate analog) 7: TCGAAGTGTATATACTATAGTATATAC;
Nucleic acid ligand (Nucleic acid polymerase substrate analog) 8: TCGGAGTGTATATACTATAGTATATACACTC;
Nucleic acid ligand (Nucleic acid polymerase substrate analog) 9: TCGGAGTGTATATACTATAGTATATACACTCC;
Nucleic acid ligand (Nucleic acid polymerase substrate analog) 10: TGAGAGTGTATATACTATAGTATATACACTCTC;
Nucleic acid ligand (Nucleic acid polymerase substrate analog) 11: GGAGAGTGTATATACTATAGTATATACACTCTCC;

In this example, a series of nucleic acid molecules with an intramolecular hairpin structure were used as nucleic acid molecules for a DNA polymerase to detect the inhibitory effect on the DNA polymerase. These nucleic acid ligands (nucleic acid polymerase substrate analogs) have 4 to all nucleotides that are complementary paired (the complementary paired bases are underlined). These nucleic acid molecules were analyzed for their blocking effect on DNA polymerase at 50°C using the method of Example 1.

It can be seen from Figure 9 that the inhibition of the nucleic acid ligands (nucleic acid polymerase substrate analogs) 2-11 on enzyme activity gradually increases and then decreases as the number of paired bases increases. When the number of paired bases is from 4 to 80 (but not limiting to this number of complementary pairs), all of the nucleic acid ligands have a certain inhibitory effect on the enzyme, but the inhibitory effects are different. Only the data with representative pattern is shown on the graph of this example. The number of paired bases is preferably from 8 to 20 in this experiment.

### Example 6: Screening of polymerase binding sequences (intermolecular)

In this example, a nucleic acid molecule modified at 3' end that can form a complementary pairing between two molecules is used as a nucleic acid ligand (nucleic acid polymerase substrate analog) for a DNA polymerase to detect the inhibitory effect on the DNA polymerase.

The following nucleic acid molecules are all modified at 3' end (dideoxy modification) (their sequences are shown in SEQ ID NOs: 12-20 successively)

| | |
|---|---|
| Nucleic acid molecule 12: | GAGGAGTTCAGTAGCATGAGCTGTGTAGACGTATATAC; |
| Nucleic acid molecule 13: | TATATACGTC; |
| Nucleic acid molecule 14: | TATATACGTCTAC; |
| Nucleic acid molecule 15: | TATATACGTCTACAC; |
| Nucleic acid molecule 16: | TATATACGTCTACACAGC; |
| Nucleic acid molecule 17: | TATATACGTCTACACAGCTC; |
| Nucleic acid molecule 18: | TATATACGTCTACACAGCTCATGC; |
| Nucleic acid molecule 19: | TATATACGTCTACACAGCTCATGCTAC; |
| Nucleic acid molecule 20: | TATATACGTCTACACAGCTCATGCTACTGAAC; |

Nucleic acid molecules 15-22 were mixed with nucleic acid molecule 14, respectively (making 10uM and 10uM respectively, and mixing) to make a mixture of nucleic acid molecules with a final concentration of 5uM as the experimental subject (i.e. nucleic acid ligands (nucleic acid polymerase substrate analogs) 12-19). According to the mixing ratio of Example 2, and referring to the addition amount of the reaction of Example 1, the change of the enzyme activity of a DNA polymerase at 50°C was tested, wherein the DNA polymerase Taq of A family and the KOD polymerase of B family with 3-5' exonucleolytic activity removed are used.

As shown in Figure 10, as the number of paired nucleotides between two nucleic acid molecules increases, the enzyme activity continuously decreases to the minimum and then increases, indicating that a certain number of paired bases are required to play the role of inhibiting the enzyme activity after part of the nucleotides of the two nucleic acid molecules are paired. Herein, 10-32 paired bases are selected to achieve the effect of inhibiting the enzyme activity, but the level of inhibition effects is different.

As shown in Figure 11, the effect of inhibition on the KOD enzyme is similar to that on Taq enzyme and the patterns are consistent, i.e., the enzyme activity decreases and then increases as the paired sequences increase. The enzyme activity can be completely blocked within a certain range.

### Example 7: Functional experimental test

The method for amplifying a target nucleic acid to be detected according to the present invention was used to amplify 18 fragments of human genome templates with different template amounts. In one group of reaction systems, nucleic acid ligand (nucleic acid polymerase substrate analog) 6 was added, and the other group of reaction systems without a nucleic acid ligand (nucleic acid polymerase substrate analog) was set as a control, and the effect of amplifying the target fragment was tested respectively.

The reaction system is:
2ul 18-site primer;
5ul buffer (Tris-HCl 8.8 30mM, NaCl 30mM, MgCl₂ 2.0 mM, BSA 1mg/ml, brij58 0.5%, proclin950 0.05%);
dATP:dTTP:dCTP:dGTP is 0.2 mM: 0.2 mM: 0.2 mM: 0.2 mM;
0.4ul Taq enzyme 10U/ul
5uM Nucleic acid ligand (Nucleic acid polymerase substrate analog) 6 (TCGAACGGGATATACTATAGTATATCC)
1ul 0.025ng/ul, 0.05ng/ul, 0.1ng/ul and 0.2ng/ul 9948 (template);
Making up to 10ul with water;
Reaction conditions: 95°C, 10 minutes; 30 cycles (95°C, 10s; 59°C, 90S single) 60°C, 10 minutes;

Figures 12 and 13 show the amplification results with and without nucleic acid ligands (nucleic acid polymerase substrate analogs) when the template amount is 0.025ng, 0.05ng, 0.1ng and 0.2ng, respectively. It can be seen that when the amplification is performed at low template concentrations (0.025ng and 0.05ng), for the system without a nucleic acid ligand (nucleic acid polymerase substrate analog), some non-specific amplification bands appear in the front circle position, which affects the accuracy of experimental determination; while for the system with the addition of nucleic acid ligands (nucleic acid polymerase substrate analogs), non-specific amplification bands are significantly reduced. This example demonstrates that non-specific amplification can be greatly reduced by the addition of nucleic acid ligands (nucleic acid polymerase substrate analogs).

### Example 8: Effect of a mixture of nucleic acid polymerase substrate analogs on reverse transcriptase activity

A mixture of two nucleic acid polymerase substrate analogs, which are the nucleic acid polymerase substrate analogs 6, 7 forming the intermolecular pairing, was used to test whether the enzyme activity could be inhibited during isothermal extension at 37°C and the enzyme activity of RT (reverse transcriptase) could be released during isothermal extension at 55°C. The mixture of nucleic acid polymerase substrate analogs in this example is a mixture of nucleic acid polymerase substrate analogs 6 and 7 in equal ratio.
Nucleic acid polymerase substrate analog 6 (SEQ ID NO: 21) TCGAACGGGACGGCTGGCTGTGTGTGT RNA with the phosphorylation modification at 3' end;
Nucleic acid polymerase substrate analog 7 (SEQ ID NO: 22)
   CCAGCCGTCC DNA with the dideoxy modification at 3' end

### Reaction system:

| Component | Volume added per aliquot ul |
|---|---|
| 5x RT buffer | 5 |
| 25mM each dNTPs | 0.2 |
| 100x SG | 0.1 |
| 100uM primer | 0.1 |
| 0.8mg/ml RNA | 0.3 |
| 25% glycerol | 5.1 |
| 200U/ul RT | 0.1 |
| 40U/ul RNase inhibitor | 0.1 |
| 6uM nucleic acid polymerase substrate analog | 1 |
| ddH₂O | 13 |

The enzyme activity is determined by a single-chain extension method using commercially available RNA and related primers. A real-time detection is performed by fluorescent quantitative method using an instrument of Roche LC480II.

Isothermal extension was carried out in the above reaction system under the following reaction conditions: (37°C, 30s) × 45 cycles and (55°C, 30s) × 45 cycles.

The enzyme activity during isothermal extension at 37°C is shown in Figure 16. As can be seen from Figure 16, the curve for the RT enzyme (reverse transcriptase) without the addition of a nucleic acid polymerase substrate analog started to bend at 14^{th} cycle during isothermal extension at 37°C, and the data from the first 14 cycles were selected for calculation. Taking the residual enzyme activity of RT enzyme without the addition of a nucleic acid polymerase substrate analog as 100% and as a reference, the residual enzyme activity of RT enzyme with the addition of the mixture of the nucleic acid polymerase substrate analogs is 16%, indicating that the addition of the mixture of the nucleic acid polymerase substrate analogs (nucleic acid polymerase substrate analogs 6 and 7) can effectively inhibit the enzyme activity of RT (reverse transcriptase).

| | Name | Increased value of enzyme activity signal | | Residual enzyme activity | |
|---|---|---|---|---|---|
| without the addition of a nucleic acid polymerase substrate analog | | | 5.16 | | 100% |
| with the addition of the mixture of the nucleic acid polymerase substrate analogs | | | 0.82 | | 16% |

The enzyme activity during isothermal extension at 55 °C is shown in Figure 17. As can be seen from Figure 17, the curves for the RT enzyme with the addition of the mixture of the nucleic acid polymerase substrate analogs and without the addition of a nucleic acid polymerase substrate analog both started to bend at the eighth cycle and reached the highest signal value of enzyme activity at 15^{th} cycle during isothermal extension at 55 °C, indicating that the addition of the mixture of the nucleic acid polymerase substrate analogs (nucleic acid polymerase substrate analogs 6 and 7) could completely (100%) release the enzyme activity of RT (reverse transcriptase).

### Example 9: Effect of the nucleic acid polymerase substrate analogs on BST DNA polymerase (DNA Polymerase) activity

In this example, the nucleic acid polymerase substrate analog 8 was used to test whether the enzyme activity could be inhibited during isothermal extension at 45 °C and the enzyme activity of BST DNA polymerase could be released during isothermal extension at 65 °C.
Nucleic acid polymerase substrate analog 8 (SEQ ID NO: 23) TTGATGACTGATCATGCATGATCAGTC

### Reaction system:

| | Component | Volume added per aliquot ul |
|---|---|---|
| | 10× bufferA | 2.5 |
| | 1M MgCl₂ | 0.125 |
| | 25mM each dNTP | 0.2 |
| | 100x SG | 0.4 |
| | 100uM primer | 0.1 |
| | 0.73mg/ml DNA | 0.45 |
| | 100U/ul BST | 0.05 |
| 2uM nucleic acid polymerase substrate analog | | 1 |
| | ddH₂O | 20.175 |

The enzyme activity is determined by a single-chain extension method using commercially available DNA and related primers. A real-time detection is performed by fluorescent quantitative method using an instrument of Roche LC480II.

Isothermal extension was carried out in the above reaction system under the following reaction conditions: (45°C, 2s) × 99 cycles and (65°C, 2s) × 99 cycles.

The enzyme activity during isothermal extension at 45 °C is shown in Figure 18. As can be seen from Figure 18, the curve for the BST enzyme without the addition of nucleic acid polymerase substrate analog 8 started to bend at 48^{th} cycle during isothermal extension at 45 °C, and the data from the first 48 cycles were selected for calculation. Taking the residual enzyme activity of BST enzyme without the addition of nucleic acid polymerase substrate analog 8 as 100% and as a reference, the residual enzyme activity of BST enzyme with the addition of nucleic acid polymerase substrate analog 8 is 8%, indicating that nucleic acid polymerase substrate analog 8 could effectively inhibit the activity of BST enzyme.

| Name | Increased value of enzyme activity signal | | Residual enzyme activity | |
|---|---|---|---|---|
| without the addition of a nucleic acid polymerase substrate analog | | 135 | | 100% |
| with the addition of the nucleic acid polymerase substrate analog | | 11 | | 8% |

The enzyme activity during isothermal extension at 65°C is shown in Figure 19. As can be seen from Figure 19, the curves for the BST enzyme with the addition of nucleic acid polymerase substrate analog 8 and without the addition of nucleic acid polymerase substrate analog 8 both started to bend at the eighth cycle and almost eached the highest signal value of enzyme activity at 18^{th} cycle during isothermal extension at 65°C, indicating that the addition of nucleic acid polymerase substrate analog 8 could completely (100%) release the enzyme activity of BST DNA polymerase.

### Example 10 Different effects of different nucleic acid polymerase substrate analogs on TAQ enzyme activity

This example tested the inhibition and release of TAQ enzyme activity at different temperatures (30°C, 40°C, 50°C, 60°C and 70°C) using nucleic acid polymerase substrate analogs 1 and 2, respectively.
Nucleic acid polymerase substrate analog 1 (SEQ ID NO: 24) TCGAACGGTATATATATTAATATATATATAC
Nucleic acid polymerase substrate analog 2 (SEQ ID NO: 25) TCGAACGGATTACAGCTGTAATC
Nucleic acid polymerase substrate analogs 1 and 2 are both dideoxy-modified at 3' end.

### I. Comparison of inhibition and release of TAQ enzyme activity

### Reaction system:

| Component | Volume added per aliquot ul |
|---|---|
| 10x bufferA | 2.5 |
| 1M MgCl₂ | 0.125 |
| 25mM each dNTPs | 0.2 |
| 100x SG | 0.4 |
| 100uM primer | 0.1 |
| 0.73mg/ml DNA | 0.45 |
| 5U/ul TAQ enzyme | 1 |
| 5uM nucleic acid polymerase substrate analog 1/ 5uM nucleic acid polymerase substrate analog 2/ddH₂O | 1 |
| ddH₂O | 19.225 |

Reaction conditions: (30/40/50/60/70°C, 30s) × 30 cycles.

The inhibition and release of enzyme activity of nucleic acid polymerase substrate analogs 1 and 2 are different under isothermal conditions at different temperatures. The enzyme activities under isothermal extension at different temperatures are shown in Figures 20-24, respectively. As can be seen from Figures 20-24, nucleic acid polymerase substrate analogs 1 and 2 inhibited the release of TAQ enzyme activity at both 30°C and 40°C. The TAQ enzyme with the addition of nucleic acid polymerase substrate analog 2 started to release enzyme activity at 50°C, and could completely release enzyme activity at 60°C and above. While nucleic acid polymerase substrate analog 1 started to release enzyme activity at 60°C and could completely release enzyme activity at 70°C.

### Example 11 Comparison of functional tests between TAQ enzyme modified by a mixture of two nucleic acid polymerase substrate analogs and TAQ enzyme modified by a single nucleic acid polymerase substrate analog

### Experimental method:

Nucleic acid polymerase substrate analog 1, nucleic acid polymerase substrate analog 2, and a mixture of nucleic acid polymerase substrate analogs 1 and 2 in equal molar ratio were mixed with TAQ enzyme respectively to perform PCR amplification. The amplification effect of the enzyme was compared.

### Reaction system:

| Component | Volume added per aliquot ul |
|---|---|
| 5x Mix1 buffer | 2 |
| 5x NH6A | 2 |
| M2 (0.03125/0.0625/0.125ng/ul) | 1 |
| NU-TAQ 8U/ul | 1 |
| ddH₂O | 4 |

### Reaction conditions:

95°C, 1min; (95°C, 10s; 59°C, 1min; 72°C, 20s) × 29 cycles; 60°C, 10min

Figures 25-27 show the amplification results of human genome M2 with template amounts of 0.03125ng, 0.0625ng, and 0.125ng respectively, with the addition of the different nucleic acid polymerase substrate analogs. As can be seen from Figures 25-27, in the amplification test of the system involving a TAQ enzyme modified by a single nucleic acid polymerase substrate analog, the non-specific amplification of small fragments was significantly more than that in the amplification test of the system involving a TAQ enzyme modified by a mixture of two nucleic acid polymerase substrate analogs, which is shown by the fact that the non-specific amplification bands in the front circle for the amplification with a single nucleic acid polymerase substrate analog are significantly more than that for the amplification with two nucleic acid polymerase substrate analogs. Therefore, the effect of an enzyme modified by a mixture of nucleic acid polymerase substrate analogs is better than that of an enzyme modified by a single nucleic acid polymerase substrate analog.

### Example 12 Results of PCR amplification by mixing a single nucleic acid polymerase substrate analog, a mixture of two nucleic acid polymerase substrate analogs and a mixture of three nucleic acid polymerase substrate analogs with Taq enzymes at low temperature and normal atmospheric temperature

Nucleic acid polymerase substrate analog 1, a mixture of nucleic acid polymerase substrate analogs 1 and 2, and a mixture of nucleic acid polymerase substrate analogs 1, 2 and 3 were mixed with Taq enzyme respectively to perform PCR amplification. The amplification effects of the enzyme were compared. The nucleic acid polymerase substrate analogs were modified at the last base at 3' end and have the dideoxy modification at 3' end in this example. Taq DNA polymerase was mixed with the nucleic acid polymerase substrate analog 1 as a control. The nucleic acid polymerase substrate analog 2 and the nucleic acid polymerase substrate analog 3 were mixed with the enzyme respectively to prepare an enzyme amount of 4U for the test. The total concentration of a mixture of nucleic acid polymerase substrate analogs 1 and 2 was 1U of the enzyme plus 3um (3umol/L) of the nucleic acid polymerase substrate analog 2 based on the control; the total concentration of a mixture of nucleic acid polymerase substrate analogs 1, 2 and 3 was 1U of the enzyme plus 3um (3umol/L) of nucleic acid polymerase substrate analog 2 and 3um (3umol/L) of nucleic acid polymerase substrate analog 3 based on the control, respectively.
Nucleic acid polymerase substrate analog 1 TCGAACGGTATATATATTAATATATATATAC
Nucleic acid polymerase substrate analog 2 TCGAACGGATTACAGCTGTAATC
Nucleic acid polymerase substrate analog 3 (SEQ ID NO: 26) TCGAACGGCTACAGCTGTAGC

### Reaction conditions and the amounts added are:

NH25: 95°C, 1min; (95°C, 10s; 59°C, 1min; 72°C, 20s)×29 cycles; 60°C, 10min

| Component | Volume added per aliquot ul |
|---|---|
| 5x Mix1 buffer | 2 |
| 5x NH25 | 2 |
| M2 (0.03125/0.0625/0.125ng/ul) | 1 |
| NU-TAQ 12 4U/ul | 2 |
| ddH₂O | 3 |

Figure 28-30 shows the results of the test after leaving the reaction mixture at 4°C for one day. From the results of the test, it can be seen that the amplification of DNA at different concentrations obviously got worse at template concentrations of 0.03125ng, 0.0625ng and 0.125ng without the addition of the nucleic acid polymerase substrate analog 2 or without the addition of the nucleic acid polymerase substrate analogs 2 and 3, which is shown by the appearance of some non-specific amplification bands in the front circle that could not even be typed and thus affected the accuracy of experimental determination; while in the system with the addition of nucleic acid polymerase substrate analog 2 or nucleic acid polymerase substrate analogs 2 and 3, non-specific amplification bands were significantly reduced. This example demonstrates that the addition of a mixture of two or three nucleic acid polymerase substrate analogs can greatly reduce non-specific amplification at low temperatures.

Figures 31-33 are the results of direct testing without placing the reaction mixture. As can be seen from the test results, at template concentrations of 0.03125ng, 0.0625ng and 0.125ng, the addition of the nucleic acid polymerase substrate analog 2 or the addition of the nucleic acid polymerase substrate analogs 2 and 3 has no effect on the normal test and the typing can be performed correctly.

### Example 13 Results of PCR amplification by mixing a mixture of 4 or 5 nucleic acid polymerase substrate analogs with Taq enzymes at low temperature

A mixture of nucleic acid polymerase substrate analogs 1, 2, 3, and 4, and a mixture of nucleic acid polymerase substrate analogs 1, 2, 3, 4 and 5, were mixed with the enzyme respectively to perform PCR amplification. The amplification effects of the enzyme were compared. The nucleic acid polymerase substrate analogs were modified at the last base at 3' end and have the dideoxy modification at 3' end in this example.

| | |
|---|---|
| Nucleic acid polymerase substrate analog 1 | TCGAACGGTATATATATTAATATATATATAC |
| Nucleic acid polymerase substrate analog 2 | TCGAACGGATTACAGCTGTAATC |
| Nucleic acid polymerase substrate analog 3 | TCGAACGGCTACAGCTGTAGC |
| Nucleic acid polymerase substrate analog 4 | TCGAACGGGATATATCC (SEQ ID NO: 27) |
| Nucleic acid polymerase substrate analog 5 | TCGAACGGGTATACC (SEQ ID NO: 28) |

The experimental method was the same as that of Example 12.

Figure 34 shows the results of the test after leaving the reaction mixture at 4°C for one day without the addition of the nucleic acid polymerase substrate analogs. As can be seen from the test results in Figure 34, at template concentrations of 0.03125ng, 0.0625ng and 0.125ng, the amplification effect becomes worse and the typing cannot be performed correctly without the addition of a nucleic acid polymerase substrate analog.

Figure 35 shows the results of the test after addition of a mixture of nucleic acid polymerase substrate analogs 1, 2, 3, and 4 and being left at 4°C for one day. As can be seen from the test results in Figure 35, at template concentrations of 0.03125ng, 0.0625ng and 0.125ng, the addition of four nucleic acid polymerase substrate analogs has no effect on the normal test and the typing can be performed correctly.

Figure 36 shows the results of the test after addition of a mixture of nucleic acid polymerase substrate analogs 1, 2, 3, 4 and 5 and being left at 4°C for one day. As can be seen from the test results in Figure 36, at template concentrations of 0.03125ng, 0.0625ng and 0.125ng, the addition of five nucleic acid polymerase substrate analogs has no effect on the normal test and the typing can be performed correctly.

This example demonstrates that the addition of mixtures of four or five nucleic acid polymerase substrate analogs can greatly reduce non-specific amplification at low temperatures.

The above-described contents are only the preferred embodiments of the present invention, and it should be pointed out that, for those skilled in the art, several improvements and modifications can be made without departing from the principles of the present invention, and these improvements and modifications should be regarded as the protection scope of the present invention.

## Claims

1. A nucleic acid ligand (nucleic acid polymerase substrate analog), **characterized in that** the nucleic acid ligand is a single nucleic acid molecule or nucleic acid molecule analog which forms intramolecular complementary pairing, or a single or two nucleic acid molecules or nucleic acid molecule analogs which form intermolecular complementary pairing; the nucleic acid ligand has a modification at 3' end, which inhibits its extension; the nucleic acid ligand forms a stable structure with a nucleic acid polymerase when the temperature is maintained or below a certain temperature, and the enzyme activity of the nucleic acid polymerase is inhibited at this time, and when the temperature is higher than said certain temperature, the nucleic acid polymerase detaches from the nucleic acid ligand to exert its activity.

2. The nucleic acid ligand according to claim 1, **characterized in that** the certain temperature is a temperature at which the nucleic acid polymerase exerts its activity.

3. The nucleic acid ligand according to claim 1, **characterized in that** the number of the complementary pairing is 8-35.

4. The nucleic acid ligand according to claim 1, **characterized in that** the modification is a dideoxy modification, a phosphorylation modification or an amino modification.

5. The nucleic acid ligand according to claim 1, **characterized in that** the nucleic acid polymerase is a DNA polymerase or a RNA polymerase.

6. The nucleic acid ligand according to claim 5, **characterized in that** the DNA polymerase is selected from the group consisting of the polymerases of Family A and Family B.

7. The nucleic acid ligand according to claim 5, **characterized in that** the RNA polymerase is selected from the group consisting of reverse transcriptases of the AMV family or the MMI,V family.

8. Use of the nucleic acid ligand according to any one of claims 1 to 7 in nucleic acid amplification, in the preparation of a nucleic acid amplification kit or in the preparation of a nucleic acid extension reaction mixture.

9. A method of nucleic acid amplification, comprising:
step 1: contacting a sample to be tested containing a target nucleic acid with the following amplification reaction reagents to form a reaction mixture;
a) primers that can hybridize to the target nucleic acid;
b) a nucleic acid polymerase;
c) the nucleic acid ligand according to any one of claims 1 to 7;
d) a nucleoside triphosphate;
step 2: heating the reaction mixture to allow the paired nucleotides of the nucleic acid ligand to dissociate into a single strand and the nucleic acid polymerase to detach from the nucleic acid ligand and exert its activity, thereby forming a primer extension product.

10. A nucleic acid amplification kit comprising the nucleic acid ligand according to any one of claims 1 to 7.

11. A nucleic acid extension reaction mixture comprising the nucleic acid ligand according to any one of claims 1 to 7, a nucleic acid polymerase, at least one primer, a nucleic acid template, and a nucleoside triphosphate.

12. A mixture of nucleic acid polymerase substrate analogs, wherein:
a. containing two or more nucleic acid polymerase substrate analogs;
b. the nucleic acid polymerase substrate analog is a single oligomeric nucleic acid molecule or nucleic acid molecule analog which forms intramolecular complementary pairing, or a single or two oligomeric nucleic acid molecules or nucleic acid molecule analogs which form intermolecular complementary pairing; the nucleic acid polymerase substrate analog forms a structure which has the characteristics of a nucleic acid polymerase substrate;
c. the nucleic acid polymerase substrate analogs are modified at 3' end, which inhibits their extension;
d. the two or more nucleic acid polymerase substrate analogs have different widths of temperature adaptation range;
e. when the temperature is maintained at or below a first temperature, the two or more nucleic acid polymerase substrate analogs are mixed with a nucleic acid polymerase and the two form a nucleic acid polymerase-substrate analog complex; at this time, the enzyme activity of the nucleic acid polymerase is significantly reduced relative to that in the absence of the nucleic acid polymerase substrate analog;
f. when the temperature is higher than the first temperature, the nucleic acid polymerase-substrate analog complex described in "e" disintegrates, and all or part of the nucleic acid polymerase activity is released.

13. A mixture of a nucleic acid polymerase and a mixture of nucleic acid polymerase substrate analogs, wherein:
a. containing two or more nucleic acid polymerase substrate analogs;
b. the nucleic acid polymerase substrate analog is a single oligomeric nucleic acid molecule or nucleic acid molecule analog which forms intramolecular complementary pairing, or a single or two oligomeric nucleic acid molecules or nucleic acid molecule analogs which form intermolecular complementary pairing; the nucleic acid polymerase substrate analog forms a structure which has the characteristics of a nucleic acid polymerase substrate, and can bind to a nucleic acid polymerase; the molecule number of each nucleic acid polymerase substrate analog is greater than the molecule number of the nucleic acid polymerase;
c. the nucleic acid polymerase substrate analogs are modified at 3' end, which inhibits their extension;
d. the two or more nucleic acid polymerase substrate analogs have different widths of temperature adaptation range;
e. when the temperature is maintained at or below a first temperature, the two or more nucleic acid polymerase substrate analogs are mixed with a nucleic acid polymerase and the two form a nucleic acid polymerase-substrate analog complex; at this time, the enzyme activity of the nucleic acid polymerase is significantly reduced relative to that in the absence of the nucleic acid polymerase substrate analog;
f. when the temperature is higher than the first temperature, the nucleic acid polymerase-substrate analog complex described in "e" disintegrates, and all or part of the nucleic acid polymerase activity is released.

14. The mixture according to claim 12 or 13, wherein:
g. when the temperature is maintained at or below a second temperature, the nucleic acid polymerase substrate analog with a wide temperature adaptation range and the nucleic acid polymerase form a nucleic acid polymerase-substrate analog complex, and the nucleic acid polymerase substrate with a narrow temperature adaptation range cannot form a nucleic acid polymerase-substrate analog complex with the nucleic acid polymerase;
the first temperature is higher than the second temperature.

15. The mixture according to any of claims 12 to 14, wherein there is a temperature difference between the first temperature and the second temperature, which is greater than or equal to 5 °C.

16. The mixture according to any one of claims 12 to 15, wherein the width of the temperature adaptation range of the nucleic acid polymerase substrate analog is related to the number of its intramolecular or intermolecular complementary paired bases;
preferably, when the temperature is maintained at or below the second temperature, the nucleic acid polymerase substrate analog having less complementary paired bases and the nucleic acid polymerase form a nucleic acid polymerase-substrate analog complex, but the nucleic acid polymerase substrate analog having more complementary paired bases cannot form a nucleic acid polymerase-substrate analog complex with the nucleic acid polymerase.

17. The mixture according to any one of claims 12-16, wherein, the number of complementary paired bases is from 8 to 35;
preferably, the number of complementary paired bases is 10-30;
more preferably, the number of complementary paired bases is 10-20;
further preferably, the number of intramolecular complementary paired bases is 8-20, and the number of intermolecular complementary paired bases is 10-32.

18. The mixture according to any one of claims 12 to 17, wherein the 3' end of the nucleic acid polymerase substrate analog is a non-OH group;
preferably, modifications at the 3' end of the nucleic acid polymerase substrate analog that inhibit its extension include dideoxy modifications, phosphorylation modifications, or amino modifications.

19. The mixture according to any one of claims 12-18, wherein the nucleic acid polymerase is a DNA polymerase or a RNA polymerase;
preferably, the DNA polymerase is a thermostable DNA polymerase;
the RNA polymerase is a reverse transcriptase.

20. Use of a mixture according to any one of claims 12-19 in nucleic acid amplification, in the preparation of a nucleic acid amplification kit or in the preparation of a nucleic acid extension reaction mixture.

21. A method of nucleic acid amplification, comprising:
step 1: contacting a sample to be tested containing a target nucleic acid with the following amplification reaction reagents to form a reaction mixture;
a) primers that can hybridize to the target nucleic acid;
b) a nucleic acid polymerase;
c) a mixture of nucleic acid polymerase substrate analogs;
d) a nucleoside triphosphate, a deoxynucleoside triphosphate or a mixture thereof, or a nucleoside/deoxynucleoside triphosphate analog;
step 2: heating the reaction mixture to allow the paired nucleotides of the nucleic acid polymerase substrate analog to dissociate into a single strand and the nucleic acid polymerase to detach from the nucleic acid polymerase substrate analog and exert its activity, thereby forming a primer extension product.

22. A nucleic acid amplification kit comprising the mixture according to any one of claims 12-19.

23. A nucleic acid extension reaction mixture comprising the mixture according to any one of claims 12-19, optionally a nucleic acid polymerase, at least one primer, a nucleic acid template; and a nucleoside triphosphate, a deoxynucleoside triphosphate or a mixture thereof, or a nucleoside/deoxynucleoside triphosphate analog.
